# EUROPEAN PATENT APPLICATION

(11) **EP 2 062 972 A1**
(43) Date of publication of application: **27.05.2009**
(21) Application number: 07807280.8
(22) Date of filing: 13.09.2007
(51) Int. Cl.: C12N 7/08, A61K 39/175, A61K 39/23, A61K 39/235, A61P 31/12, C12N 15/09

(54) **NOVEL VACCINE FOR DOG**

(30) Priority: 13.09.2006 JP 2006248522
(71) Applicant: Nippon Zenyaku Kogyo Co., Ltd., Koriyama-shi, Fukushima 963-0196 (JP)
(72) Inventor: OKAZAKI, Masayuki, Koriyama-shi Fukushima 963-0196 (JP); YOSHIMURA, Takahiro, Koriyama-shi Fukushima 963-0196 (JP); TSUKUI, Toshihiro, Koriyama-shi Fukushima 963-0196 (JP); IWABUCHI, Shigehiro, Koriyama-shi Fukushima 963-0196 (JP)
(74) Representative: Denison, Christopher Marcus
(86) International application number: PCT/JP2007/067873
(87) International publication number: WO 2008/032796

(57) **Abstract**

This invention provides vaccines against canine distemper virus infections, canine adenovirus type 2 infections, and canine parvovirus infections that can be orally administered. The invention also provides the attenuated canine distemper virus strain obtained by adapting the canine distemper virus 95-54 strains in cultured cells to attenuate the same, the attenuated canine adenovirus type 2 strain obtained by adapting the canine adenovirus type 2 F1 strain in cultured cells to attenuate the same, and an attenuated canine parvovirus strain obtained by adapting the canine parvovirus F3 strain in cultured cells to attenuate the same.

## Description

### Technical Field

The present invention relates to vaccines against canine infections, such as canine distemper virus infections, canine adenovirus type 2 infections, and canine parvovirus infections.

### Background Art

Canine distemper viruses (CDVs) are viruses that cause acute systemic canine diseases exhibiting symptoms, such as onset of fever, central nervous system manifestations, respiratory symptoms, or diarrhea. Puppies are highly likely to be infected with such diseases, and the mortality is high. Even if dogs recover from infections, they may develop aftereffects, such as central nervous system manifestations and viruses that remain latent in their bodies, and they may suffer relapses several months to several years later. Examples of representative symptoms include respiratory symptoms, such as sneezing, runny nose, and cough. In addition, digestive symptoms, neurological symptoms, and other symptoms are observed. Examples of digestive symptoms include vomition, watery diarrhea, and hemorrhagic stool, and examples of neurological symptoms include exaltation, epileptiform attack, tic, pelvic limb paralysis, and dynamic ataxia.

Canine adenovirus type 2 (CAV2) is known to cause canine infectious tracheobronchitis. Acute and chronic Infectious diseases exhibiting respiratory symptoms, such as coughing and sneezing, except for canine distemper, are collectively referred to as "kennel cough," and canine adenovirus type 2 is a virus that causes kennel cough. CAV2-vaccines are known to have antigenicity in common with canine adenovirus type 1 (CAV1) that causes infectious canine hepatitis.

Canine parvovirus (CPV) causes digestive symptoms characterized mainly by pungent diarrhea, and infected puppies develop myocarditis and eventually die. Parvovirus is highly resistant to antiseptic solutions, outdoor environmental conditions, and so on. A large amount thereof can be shed in the stool of infected dogs, viruses excreted from bodies do not die immediately, but survive in nature for several years.

Thus, such 3 types of virus infections are very serious, and a high mortality rate in the event of infection of puppies therewith, in particular, has been problematic.

Vaccines against such virus infections have already been developed (see JP Patent No. 3040157 and JP Patent Publication (kohyo) No. 2004-501979 A). In addition to plain vaccines against viruses of such infections, two-way vaccines, three-way vaccines, and the like have been used.

Currently available vaccines are administered by injection, and the likelihood of side-effects, such as anaphylactic shock or hypersensitivity, resulting therefrom is reported (see Gaskell. R. M. et al., Vet, Rec. 150, 126-34, 2002). Also, such vaccination has been problematic also in terms of pain or stress imposed on dogs at the time of injection. When such vaccines are used in the form of combination vaccines, interference among viruses may disadvantageously suppresses virus growth, and the antibody titer against the viruses is not elevated. In particular, combination vaccines against distemper viruses and parvoviruses are likely to cause virus interference (GEMMA T., et al., J. Vet. Med., Sci., 57 (3): 535-537, 1995).

### Disclosure of the invention

The present invention provides orally administrable vaccines against canine distemper virus infections, canine adenovirus type 2 infections, and canine parvovirus infections.

Many conventional vaccines against canine distemper virus infections, canine adenovirus type 2 infections, and canine parvovirus infections have been administered via injection. When such vaccines were administered via injection, side-effects, such as allergic symptoms, were likely to develop disadvantageously.

Also, newborn puppies have received maternal antibodies from their mothers. If puppies were subjected to vaccination while there were still adequate maternal antibodies presented in their bodies, accordingly, the maternal antibodies would disadvantageously neutralize the vaccines, and puppies would not be immunized due thereto. Maternal antibodies, which were passively acquired within 2 days after birth via colostrum, begin to decrease gradually soon after birth, and substantially all maternal antibodies generally disappear by about 12 to 14 weeks after birth, although there are individual differences. It is said to be ideal to vaccinate puppies immediately after maternal antibodies are gone. In practice, however, puppies are often afflicted with canine distemper virus infections, canine adenovirus type 2 infections, and canine parvovirus infections when maternal antibodies decrease. Thus, it has been impossible to completely protect puppies from such infections.

The present inventors established novel strains by attenuating high virulent field isolates of the canine distemper virus, canine adenovirus type 2, and canine parvovirus strains. Conventional vaccines were administered via injection, and dogs could not be immunized via oral administration of conventional vaccines. However, the present inventors discovered that the novel strains of present invention might immunize dog via gastrointestinal or transmucosal administration such as oral or nasal administration while avoiding side effects caused by injection administration. Also, the strains that the present inventors had established exhibited elevated antibody titers against all the mixed strains without causing interference among viruses, even when they were administered in the form of combination vaccines. Further, they also discovered that oral administration of such vaccines to puppies could immunize puppies without causing neutralization by maternal antibodies acquired from mothers. This has led to the completion of the present invention.

Specifically, the present invention is as follows.
[1] A canine parvovirus F3 strain having a VP2 gene comprising the nucleotide sequence as shown in SEQ ID NO: 1 or a nucleotide sequence derived from the nucleotide sequence as shown in SEQ ID NO: 1 by deletion, substitution, or addition of one or several nucleotides.
[2] The canine parvovirus F3 strain according to [1], which is the strain of any of the viruses (1) to (7) below:
   (1) a virus having a genome comprising DNA containing the nucleotide sequence as shown in SEQ ID NO: 26;
   (2) a virus having a genome comprising DNA containing a nucleotide sequence consisting of 500, 750, 1000, 1250, 1500, 1750, 2000, 2250, 2500, 2750, 3000, 3250, 3500, 3750, or 4000 continuous nucleotides of the nucleotide sequence as shown in SEQ ID NO: 26;
   (3) a virus having a genome comprising DNA containing a nucleotide sequence consisting of nucleotides from 188, 200, or 630 to 4000 or 4017 of the nucleotide sequence as shown in SEQ ID NO: 26;
   (4) a virus having a genome comprising DNA containing a nucleotide sequence consisting of nucleotides from 188, 200, or 630 to 480 of the nucleotide sequence as shown in SEQ ID NO: 26;
   (5) a virus having a genome comprising DNA containing a nucleotide sequence consisting of nucleotides from 500 to 4000 or 4017 of the nucleotide sequence as shown in SEQ ID NO: 26;
   (6) a virus having a genome comprising DNA that hybridizes under stringent hybridization conditions to DNA comprising a sequence complementary to DNA consisting of the nucleotide sequence of the genome according to any of (1) to (5) above; and
   (7) a virus having a genome consisting of DNA having 95% or higher homology to DNA consisting of the nucleotide sequence of the genome according to any of (1) to (5) above.
[3] A canine adenovirus type 2 F1 strain having an LITR E1A2 gene consisting of the nucleotide sequence as shown in SEQ ID NO: 5 or a nucleotide sequence derived from the nucleotide sequence as shown in SEQ ID NO: 5 by deletion, substitution, or addition of one or several nucleotides and having an RITR gene consisting of the nucleotide sequence as shown in SEQ ID NO: 7 or a nucleotide sequence derived from the nucleotide sequence as shown in SEQ ID NO: 7 by deletion, substitution, or addition of one or several nucleotides.
[4] The canine adenovirus type 2 F1 strain deposited at the European Collection of Cell Cultures (ECACC) under Accession Number 07071601.
[5] A canine distemper virus 95-54 strain having an H gene consisting of the nucleotide sequence as shown in SEQ ID NO: 9 or a nucleotide sequence derived from the nucleotide sequence as shown in SEQ ID NO: 9 by deletion, substitution, or addition of one or several nucleotides and having an F gene consisting of the nucleotide sequence as shown in SEQ ID NO: 12 or a nucleotide sequence derived from the nucleotide sequence as shown in SEQ ID NO: 12 by deletion, substitution, or addition of one or several nucleotides.
[6] The canine distemper virus 95-54 strain deposited at the European Collection of Cell Cultures (ECACC) under Accession Number 07071602.
[7] An attenuated canine parvovirus strain obtained by adapting the canine parvovirus F3 strain according to [1] or [2] in cultured cells to attenuate the same.
[8] The attenuated canine parvovirus strain according to [7], which has a nucleotide sequence derived from the nucleotide sequence of the VP2 gene as shown in SEQ ID NO: 1 by mutation of one or a plurality of, and preferably all of, nucleotides 885, 886, 892, 894, and 895.
[9] The attenuated canine parvovirus strain according to [7] or [8], which has a nucleotide sequence comprising 2 nucleotides inserted into a site between nucleotides 891 and 892 and/or 100 nucleotides inserted into a site between nucleotides 901 and 902.
[10] An attenuated canine adenovirus type 2 strain obtained by adapting the canine adenovirus type 2 F1 strain according to [3] or [4] in cultured cells to attenuate the same.
[11] The attenuated canine adenovirus type 2 strain according to [10], which has a nucleotide sequence derived from the nucleotide sequence of the LITR gene as shown in SEQ ID NO: 5 by mutation of one or a plurality of nucleotides.
[12] An attenuated canine distemper virus strain obtained by adapting the canine distemper virus 95-54 strain according to [5] or [6] in cultured cells to attenuate the same.
[13] The attenuated canine distemper virus strain according to [12], which has at least 1 of mutations (1) to (3) below:
   (1) mutation of nucleotides 394 and/or 523 of the F gene as shown in SEQ ID NO: 12;
   (2) mutation of one or a plurality of, and preferably all of, nucleotides 56, 74, 78, 94, 100, 110, 116, 128, 135, 142, 156, 159, 174, 176, 182, 204, 218, 278, 306, 311, 320, 350, 374, 389, 398, 407, 416, 437, 446, 452, 456, 482, 485, 489, 495, 499, 531, 542, 548, 551, 553, 557, 558, 572, 598, 610, 614, 626, 629, 632, 647, 650, 659, 664, 680, 689, 698, 707, 716, 732, 737, 741, 753, 755, 776, 785, 788, 792, 812, 821, 850, 872, 884, 888, 914, 923, 929, 932, 935, 946, 947, 955, 957, 968, 977, 978, 983, 999, 1010, 1011, 1016, 1019, 1044, 1051, 1067, 1076, 1079, 1094, 1096, 1115, 1116, 1120, 1136, 1139, 1146, 1147, 1160, 1176, 1184, 1214, 1232, 1255, 1263, 1268, 1269, 1292, 1295, 1301, 1319, 1325, 1351, 1356, 1374, 1382, 1385, 1395, 1406, 1442, 1444, 1445, 1454, 1457, 1466, 1479, 1499, 1511, 1514, 1520, 1525, 1535, 1537, 1538, 1559, 1570, 1585, 1592, 1604, 1607, 1608, 1609, 1610, 1615, 1619, 1644, 1657, 1658, 1676, 1720, 1733, 1734, 1758, 1762, 1776, 1778, 1838, 1840, 1845, 1851, 1871, 1877, 1879, 1889, 1894, 1896, 1908, and 1911 of the H gene as shown in SEQ ID NO: 10; and
   (3) mutation of one or a plurality of, and preferably all of, nucleotides 628, 634, 644, 648, 657, 681, 683, 718, 744, 748, 823, and 1406 of the H gene as shown in SEQ ID NO: 10.
[14] A vaccine against canine virus infections comprising 1, 2, or 3 types of virus strains selected from the group consisting of the attenuated canine parvovirus strain according to any of [7] to [9], the attenuated canine adenovirus type 2 strain according to [10] or [11], and the attenuated canine distemper virus strain according to [12] or [13].
[15] A combination vaccine against canine virus infections comprising 3 types of virus strains selected from the group consisting of the attenuated canine parvovirus strain according to any of [7] to [9], the attenuated canine adenovirus type 2 strain according to [10] or [11], and the attenuated canine distemper virus strain according to [12] or [13].
[16] The combination vaccine against canine virus infections according to [14] or [15], wherein different virus strains do not interfere with each other.
[17] The combination vaccine against canine virus infections according to [16], wherein the canine distemper virus and the canine parvovirusdo not interfere with each other.
[18] The vaccine against canine virus infections according to any of [14] to [17], which is for oral administration.
[19] The vaccine against canine virus infections according to any of [14] to [18], which can immunize a puppy when it is vaccinated therewith up to 4 to 18 weeks after birth.
[20] A method for protecting a dog from virus infections comprising orally administering 1, 2, or 3 types of virus strains selected from the group consisting of the attenuated canine parvovirus strain according to any of [7] to [9], the attenuated canine adenovirus type 2 strain according to [10] or [11], and the attenuated canine distemper virus strain according to [12] or [13] to a dog.
[21] The method for protecting a dog from virus infections according to [20], wherein different virus strains do not interfere with each other.
[22] The method for protecting a dog from virus infections according to [20] or [21], wherein the canine distemper virus and the canine parvovirusdo not interfere with each other.
[23] The method for protecting a dog from virus infections according to any of [20] to [22] comprising vaccination of a 4- to 18-week old puppy.

### Effects of the Invention

The novel attenuated canine distemper virus strain, canine adenovirus strain, and canine parvovirus strain of the present invention can immunize a dog via oral administration thereof. Oral administration can reduce the time and cost required for vaccination. Furthermore, many dogs can be immunized at once in a group. Even if the virus strains are administered to puppies that still carry maternal antibodies acquired from mother dogs, such virus strains would not be neutralized and eliminated by maternal antibodies, and puppies can be immunized. Therefore, such vaccines can be administered to puppies that have high risk of canine distemper virus infections, canine adenovirus type 1 and type 2 infections, and canine parvovirus infections due to declining in of maternal antibody although the level thereof is still adequate to disadvantagely neutralize conventional vaccines. Thus, such vaccines can protect puppies from above virus infections.

This description includes part or all of the contents as disclosed in the description and/or drawings of Japanese Patent Application No. 2006-248522, which is a priority document of the present application.

### Brief Description of the Drawings

Fig. 1A shows an alignment of the nucleotide sequence of the VP2 gene of a canine parvovirus strain at the 10th passage and the nucleotide sequence of the strain at the 50th passage thereof.
Fig. 1B shows an alignment of the nucleotide sequence of the VP2 gene of a canine parvovirus strain at the 10th passage and the nucleotide sequence of the strain at the 50th passage thereof (continuation of Fig. 1A).
Fig. 1C shows an alignment of the nucleotide sequence of the VP2 gene of a canine parvovirus strain at the 10th passage and the nucleotide sequence of the strain at the 50th passage thereof (continuation of Fig. 1B).
Fig. 2A shows an alignment of the nucleotide sequence of the N gene of a canine parvovirus strain at the 10th passage and the nucleotide sequence of the strain at the 50th passage thereof.
Fig. 2B shows an alignment of the nucleotide sequence of the N gene of a canine parvovirus strain at the 10th passage and the nucleotide sequence of the strain at the 50th passage thereof (continuation of Fig. 2A).
Fig. 2C shows an alignment of the nucleotide sequence of the N gene of a canine parvovirus strain at the 10th passage and the nucleotide sequence of the strain at the 50th passage thereof (continuation of Fig. 2B).
Fig. 3A shows an alignment of the nucleotide sequence of the LITR E1A gene of a canine adenovirus type 2 strain at the 1st passage and the nucleotide sequence of the strain at the 50th passage thereof.
Fig. 3B shows an alignment of the nucleotide sequence of the LITR E1A gene of a canine adenovirus type 2 strain at the 1st passage and the nucleotide sequence of the strain at the 50th passage thereof (continuation of Fig. 3A).
Fig. 3C shows an alignment of the nucleotide sequence of the LITR E1A gene of a canine adenovirus type 2 strain at the 1st passage and the nucleotide sequence of the strain at the 50th passage thereof (continuation of Fig. 3B).
Fig. 3D shows an alignment of the nucleotide sequence of the LITR E1A gene of a canine adenovirus type 2 strain at the 1st passage and the nucleotide sequence of the strain at the 50th passage thereof (continuation of Fig. 3C).
Fig. 4 shows an alignment of the nucleotide sequence of the RITR gene of a canine adenovirus type 2 strain at the 1st passage and the nucleotide sequence of the strain at the 50th passage thereof.
Fig. 5A shows an alignment of a nucleotide sequence of a parent strain of the H gene of a canine distemper virus strain, the nucleotide sequence of the strain at the 14th passage thereof, and the nucleotide sequence of the strain at the 50th passage thereof.
Fig. 5B shows an alignment of a nucleotide sequence of a parent strain of the H gene of a canine distemper virus strain, the nucleotide sequence of the strain at the 14th passage thereof, and the nucleotide sequence of the strain at the 50th passage thereof (continuation of Fig. 5A).
Fig. 5C shows an alignment of a nucleotide sequence of a parent strain of the H gene of a canine distemper virus strain, the nucleotide sequence of the strain at the 14th passage thereof, and the nucleotide sequence of the strain at the 50th passage thereof (continuation of Fig. 5B).
Fig. 5D shows an alignment of a nucleotide sequence of a parent strain of the H gene of a canine distemper virus strain, the nucleotide sequence of the strain at the 14th passage thereof, and the nucleotide sequence of the strain at the 50th passage thereof (continuation of Fig. 5C).
Fig. 5E shows an alignment of a nucleotide sequence of a parent strain of the H gene of a canine distemper virus strain, the nucleotide sequence of the strain at the 14th passage thereof, and the nucleotide sequence of the strain at the 50th passage thereof (continuation of Fig. 5D).
Fig. 6A shows an alignment of a nucleotide sequence of a parent strain of the F gene of a canine distemper virus strain and the nucleotide sequence of the strain at the 14th passage thereof.
Fig. 6B shows an alignment of a nucleotide sequence of a parent strain of the F gene of a canine distemper virus strain and the nucleotide sequence of the strain at the 14th passage thereof (continuation of Fig. 6A).
Fig. 6C shows an alignment of a nucleotide sequence of a parent strain of the F gene of a canine distemper virus strain and the nucleotide sequence of the strain at the 14th passage thereof (continuation of Fig. 6B).
Fig. 6D shows an alignment of a nucleotide sequence of a parent strain of the F gene of a canine distemper virus strain and the nucleotide sequence of the strain at the 14th passage thereof (continuation of Fig. 6C).
Fig. 6E shows an alignment of a nucleotide sequence of a parent strain of the F gene of a canine distemper virus strain and the nucleotide sequence of the strain at the 14th passage thereof (continuation of Fig. 6D).
Fig. 7A shows an alignment of the nucleotide sequence of the H gene of a canine distemper virus strain at the 14th passage and the nucleotide sequence of the strain at the 50th passage thereof.
Fig. 7B shows an alignment of the nucleotide sequence of the H gene of a canine distemper virus strain at the 14th passage and the nucleotide sequence of the strain at the 50th passage thereof (continuation of Fig. 7A).
Fig. 7C shows an alignment of the nucleotide sequence of the H gene of a canine distemper virus strain at the 14th passage and the nucleotide sequence of the strain at the 50th passage thereof (continuation of Fig. 7B).
Fig. 8 shows transition in antibody titers after canine parvovirus inoculation.
Fig. 9 shows the results of the protection test regarding canine parvovirus infections.
Fig. 10 shows the results of the safety test regarding canine parvovirus infections.

### Best Modes for Carrying out the Invention

The present invention relates to attenuated canine distemper virus, canine adenovirus type 2 virus, and canine parvovirus strains. The present invention also relates to a vaccine against canine distemper, a vaccine against canine adenovirus type 2 infections, and a vaccine against canine parvovirus infections comprising such attenuated virus strains.

The term "attenuate" refers to reduction of virulence of a parent strain that is originally pathogenic, and an attenuated virus strain is infectious but is not pathogenic.

A parent strain to be attenuated is a virus strain isolated from a pathogenic wild-type strain (i.e., a virulent strain). A pathogenic virus strain can be isolated from a dog. As a pathogenic virus isolate to be used as a parent strain in the present invention, a canine distemper virus 95-54 strain may be used, the F1 strain may be used as a canine adenovirus type 2 strain, and the F3 strain may be used as a canine parvovirus strain. Virus strains are attenuated by subjecting the virus strains to serial subculture in adequate cultured cells for a period of time during which mutations that attenuate parent strains are accumulated to adapt such strains in the cultured cells. The term "serial subculture" refers to infection of the cells with virus strains, recovery of the offspring viruses from the host cells, and acquisition of the next generation via subsequent infection of the host cells with the offspring viruses. Examples of cultured cells that can be used for subculture for attenuation include normal African green monkey kidney epithelial cells (VERO cells), normal dog kidney cells (MDCK), established feline kidney cells (CRFK cells), marmoset B-lymphoblast cells (B95a cells), and canine cells (A72 cells). Canine distemper virus strains are subcultured with the use of VERO cells or B95a cells, with VERO cells being preferable. Canine adenovirus type 2 strains are subcultured with the use of MDCK cells or CRFK cells, with MDCK cells being preferable. Canine parvovirus strains are preferably subcultured with the use of A72 cells or CRFK cells. The attenuated virus strains of the present invention are obtained as a result of subculture for 10 passages, preferably for 50 passages, more preferably for 75 passages, and further preferably for 100 passages with the use of cultured cells. Virus strains are subcultured in the following method.

In the case of canine distemper virus strains and canine adenovirus type 2 strains
1. Cells are subcultured.
2. Medium is removed upon formation of a monolayer.
3. A virus solution is innoculated to cells.
4. Incubation at 37°C for 1 hour to allow attachment of the virus to cells.
5. Cell maintenance medium is added.
6. Cells are incubated in a CO₂ incubator at 37°C.
7. When CPE is observed, it is frozen at -80°C and then subjected to further subculture.
In the case of canine parvovirus strains
1. Cells are subcultured.
2. A virus solution is innoculated to cells.
3. Incubation at 37°C for 1 hour to allow attachment of the virus to cells.
4. When CPE is observed, it is frozen at -80°C and then subjected to further subculture.

Target virus strains from the parent strains are subjected to serial subculture to acquire mutants. A dog may be inoculated with the subcultured virulent strains, and the clinical profile of the dog may be investigated to determine whether or not the mutants of interest have been attenuated. When a given virus strain is determined to be capable of infecting a dog but incapable of causing a disease, the strain of interest can be identified as having been attenuated.

After the attenuated viruses have been isolated, the genome sequences thereof may be analyzed.

In the attenuated virus strain of the present invention, a gene associated with virus pathogenecity has been mutated and attenuated. Examples of genes associated with virus pathogenecity of the canine parvovirus strains include genes encoding VP1 (virion capsid protein 1) and VP2 (virion capsid protein 2). Examples regarding the canine distemper virus strains include F (fusion protein) and H (hemagglutinin protein) genes.

The nucleotide sequences of parent strains of the genes of 3 types of virus strains shown below (i.e., challenge strains) or the nucleotide sequences of the subcultured strains are shown in the sequence listings under the sequence identification numbers shown below.

### Canine parvovirus

VP2; subcultured for 10 passages: SEQ ID NO: 1
VP2; subcultured for 50 passages: SEQ ID NO: 2
N; subcultured for 10 passages: SEQ ID NO: 3
N; subcultured for 50 passages: SEQ ID NO: 4

### Canine adenovirus type 2

LITR E1A; subcultured for 1 passage: SEQ ID NO: 5
LITR E1A; subcultured for 50 passages: SEQ ID NO: 6
RITR; subcultured for 1 passage: SEQ ID NO: 7
RITR; subcultured for 50 passages: SEQ ID NO: 8

### Canine distemper virus

H; parent strain (challenge strain): SEQ ID NO: 9
H; subcultured for 14 passages: SEQ ID NO: 10
H; subcultured for 50 passages: SEQ ID NO: 11
F; parent strain (challenge strain): SEQ ID NO: 12
F; subcultured for 14 passages: SEQ ID NO: 13
NP; subcultured for 14 passages: SEQ ID NO: 14
NP; subcultured for 50 passages: SEQ ID NO: 15

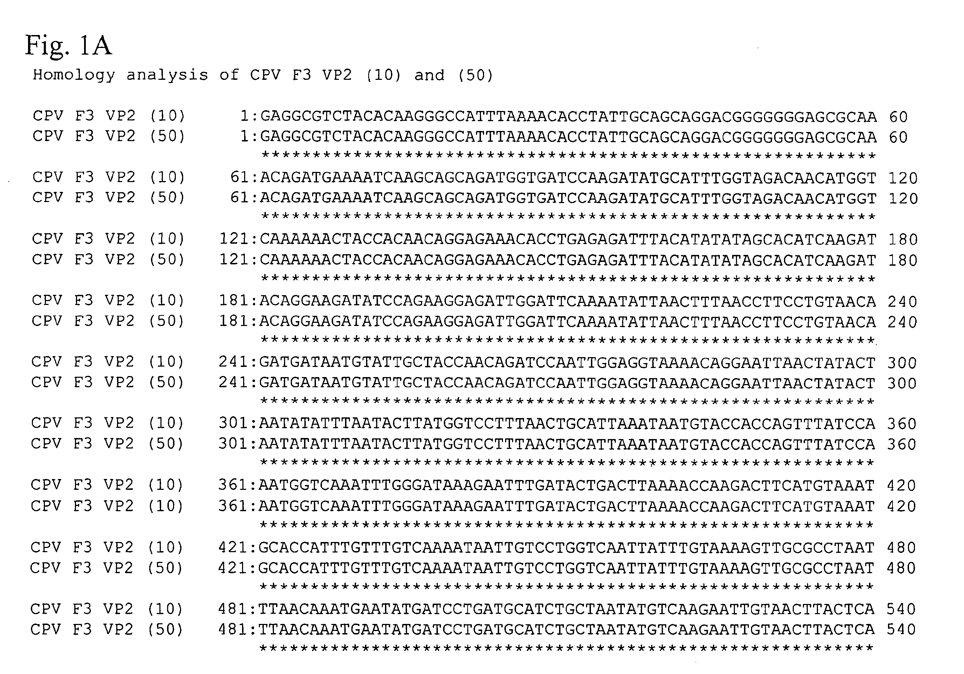

Comparison of the nucleotide sequence of the VP2 gene of the canine parvovirus strain at the 10^{th} passage and the nucleotide sequence at the 50^{th} passage thereof is shown in Figs. 1A to 1C.

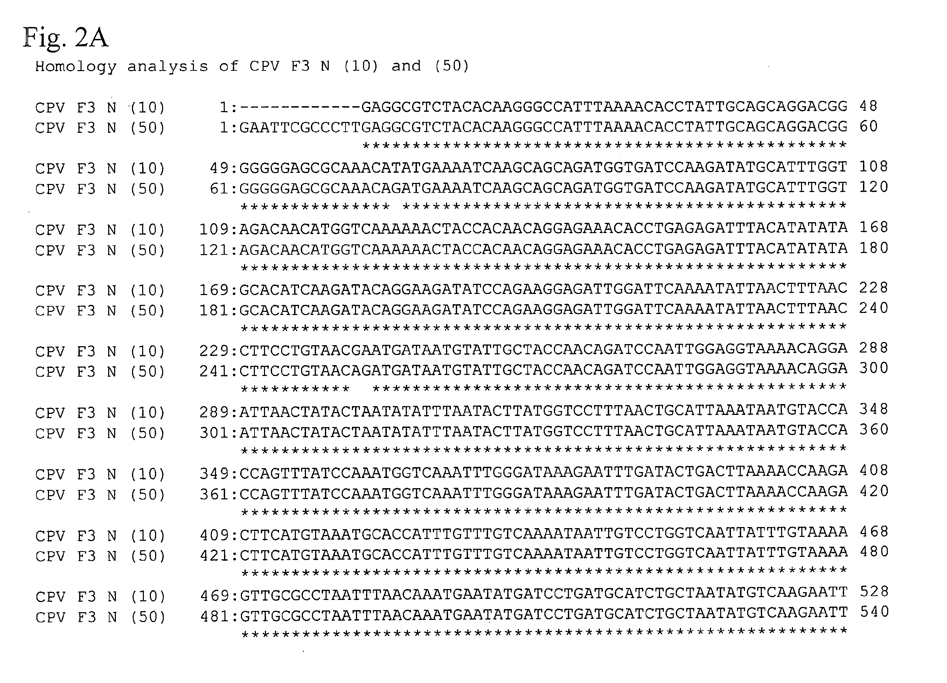

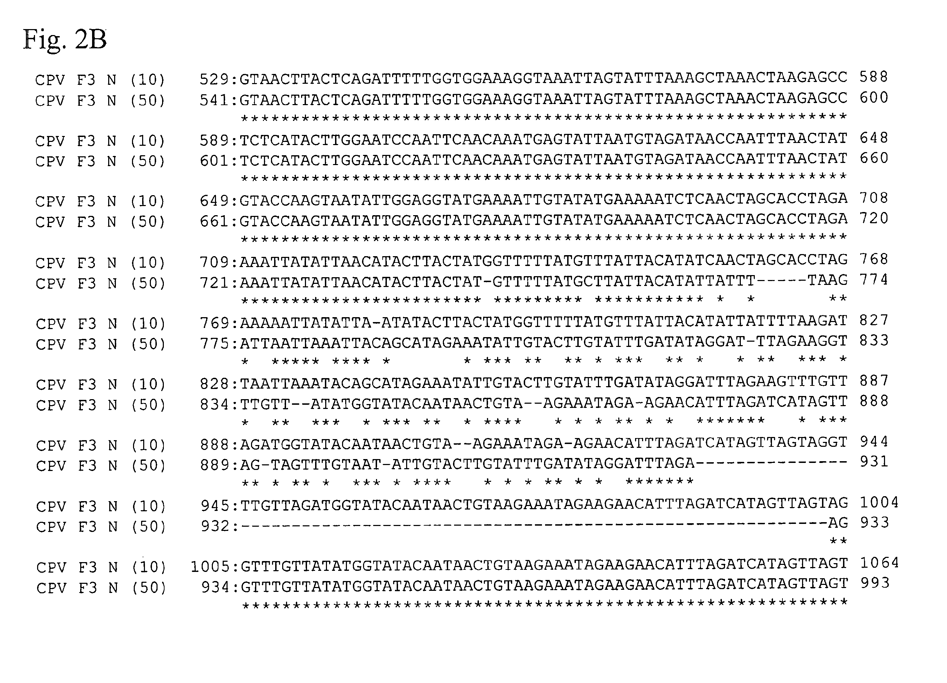

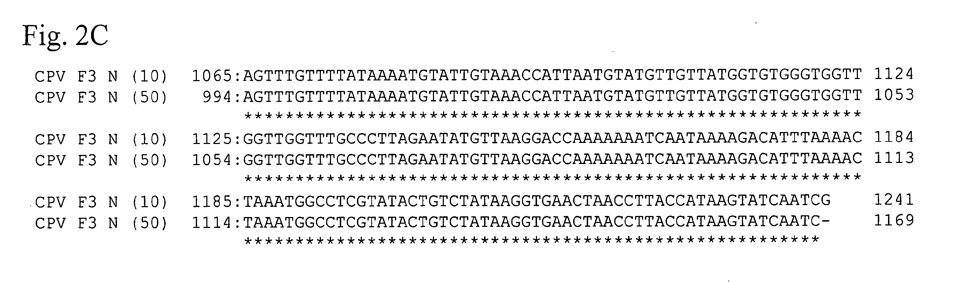

Comparison of the nucleotide sequence of the N gene of the canine parvovirus strain at the 10^{th} passage and the nucleotide sequence at the 50^{th} passage thereof is shown in Figs. 2A to 2C.

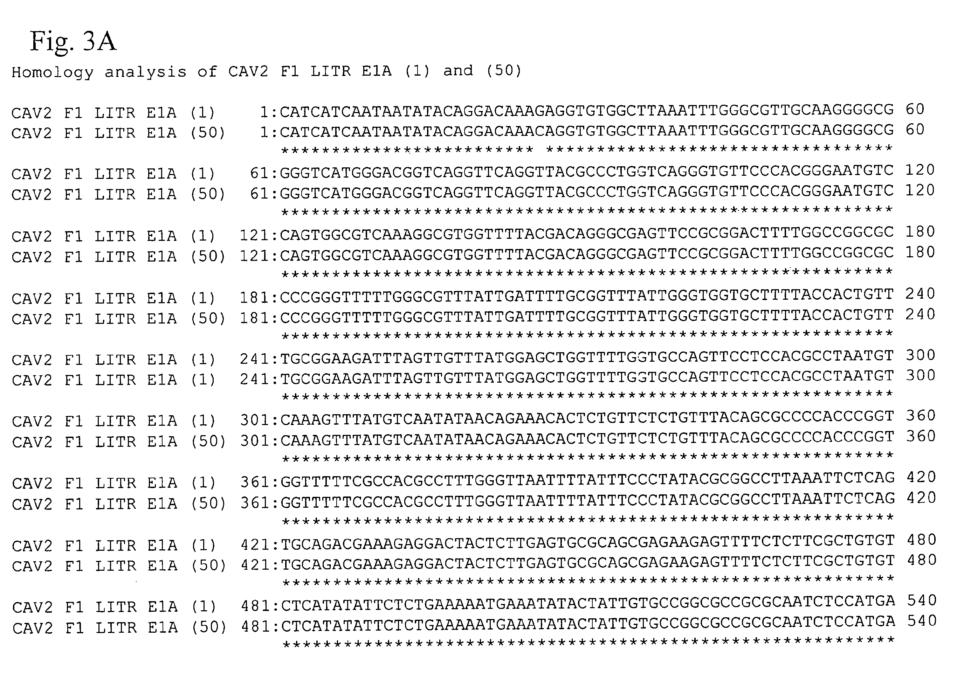

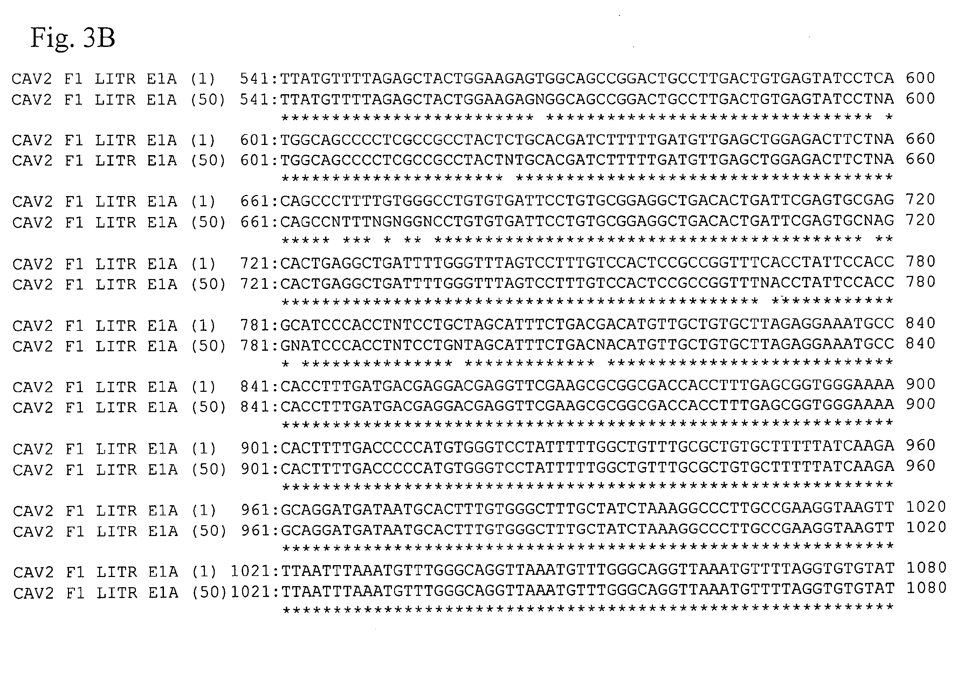

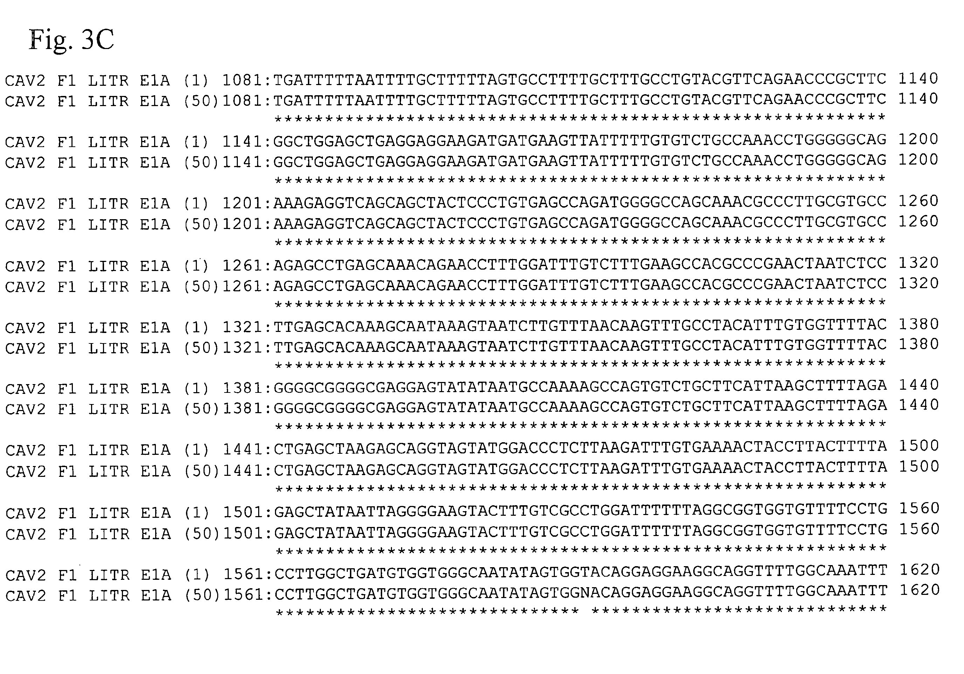

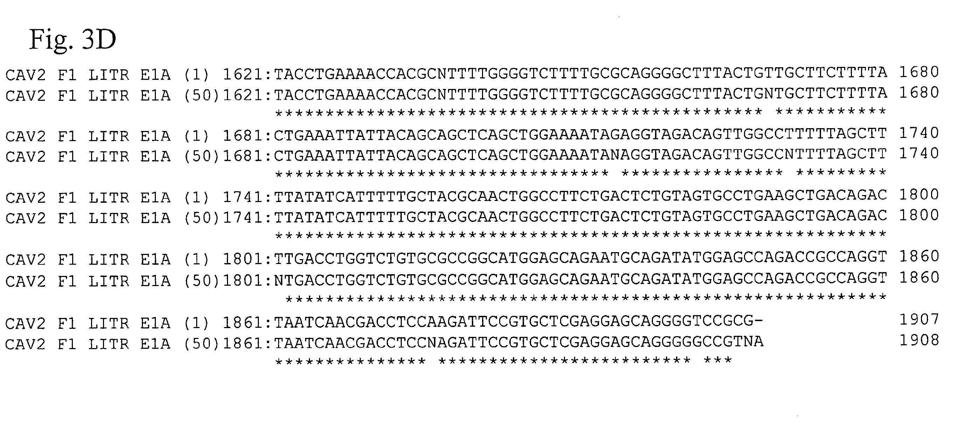

Comparison of the nucleotide sequence of the LITR E1A gene of the canine adenovirus type 2 strain at the 1^{st} passage and the nucleotide sequence at the 50^{th} passage thereof is shown in Figs. 3A to 3D.

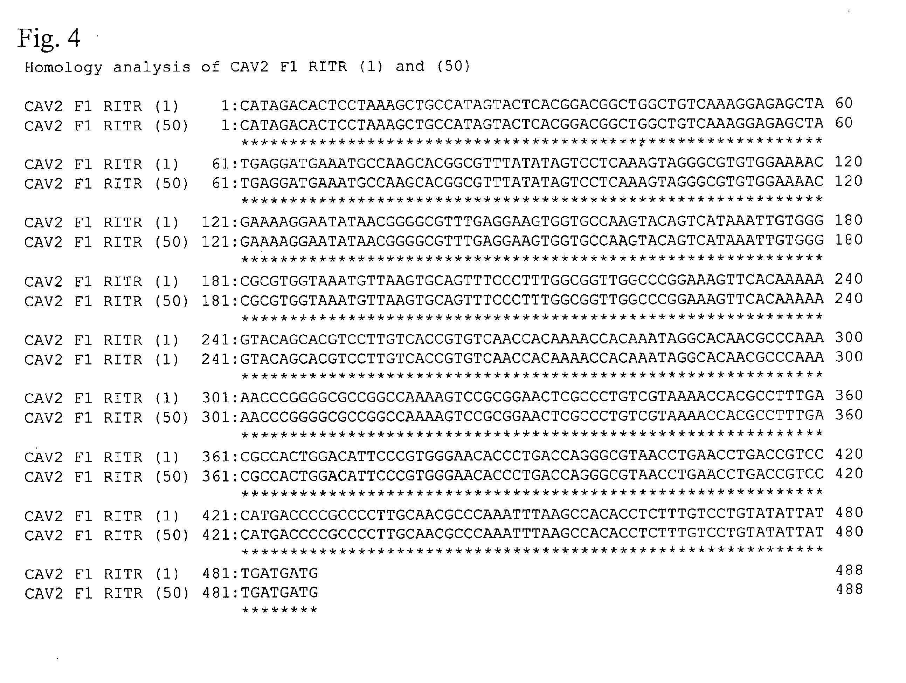

Comparison of the nucleotide sequence of the RITR gene of the canine adenovirus type 2 strain at the 1^{st} passage and the nucleotide sequence at the 50^{th} passage thereof is shown in Fig. 4.

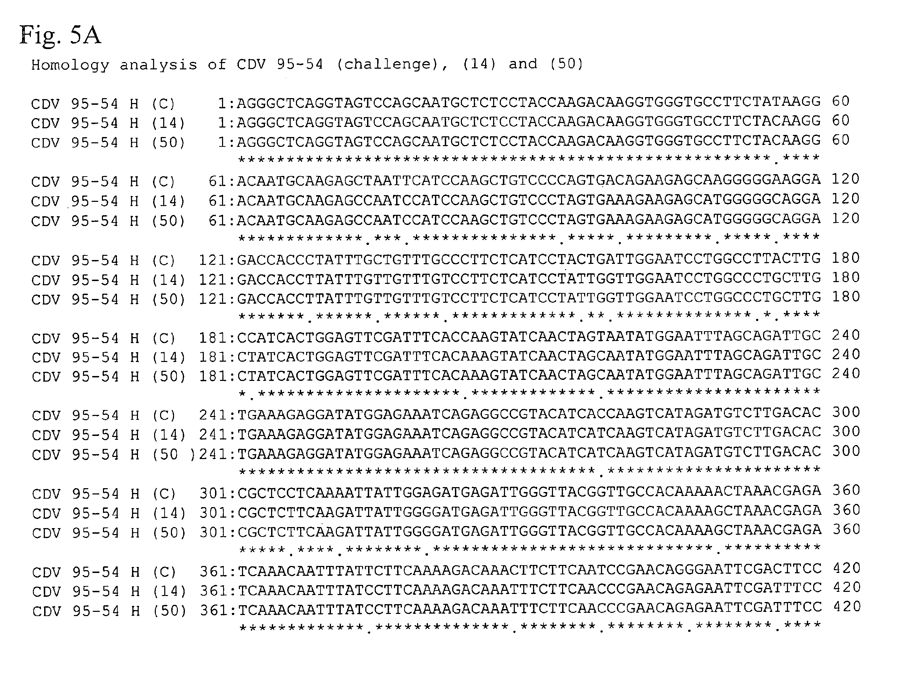

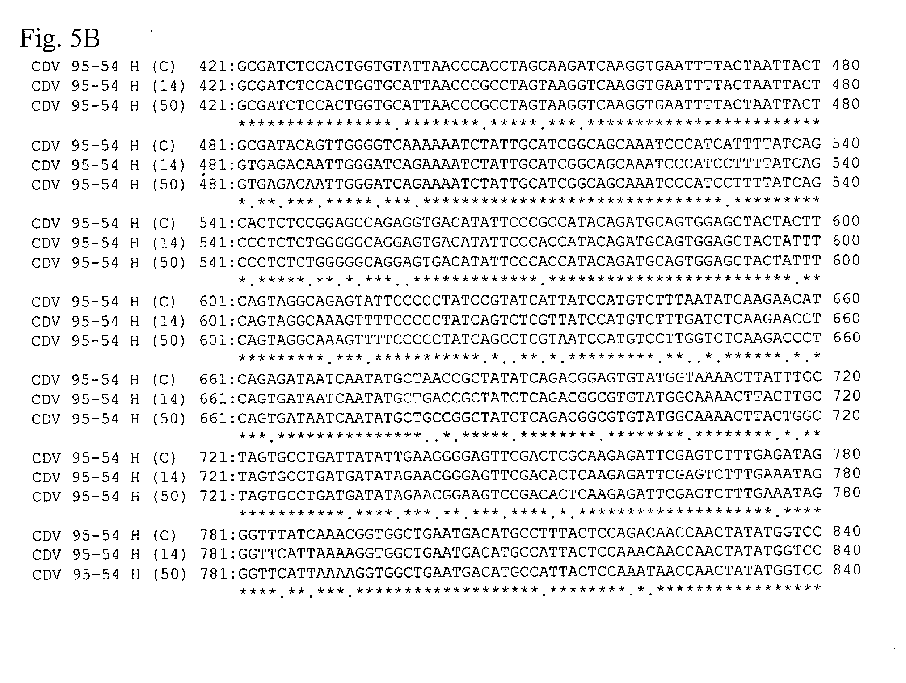

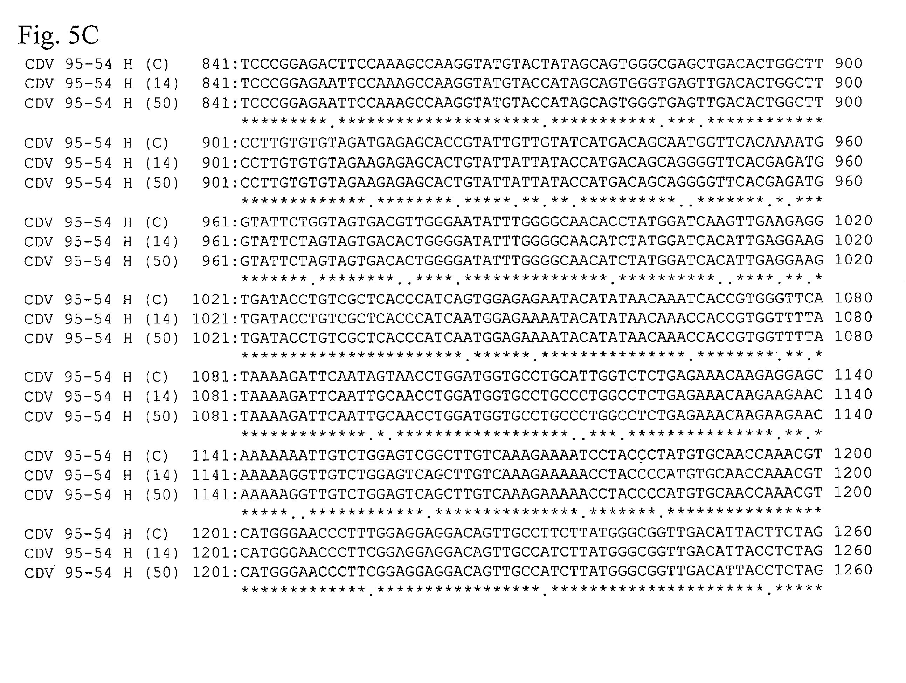

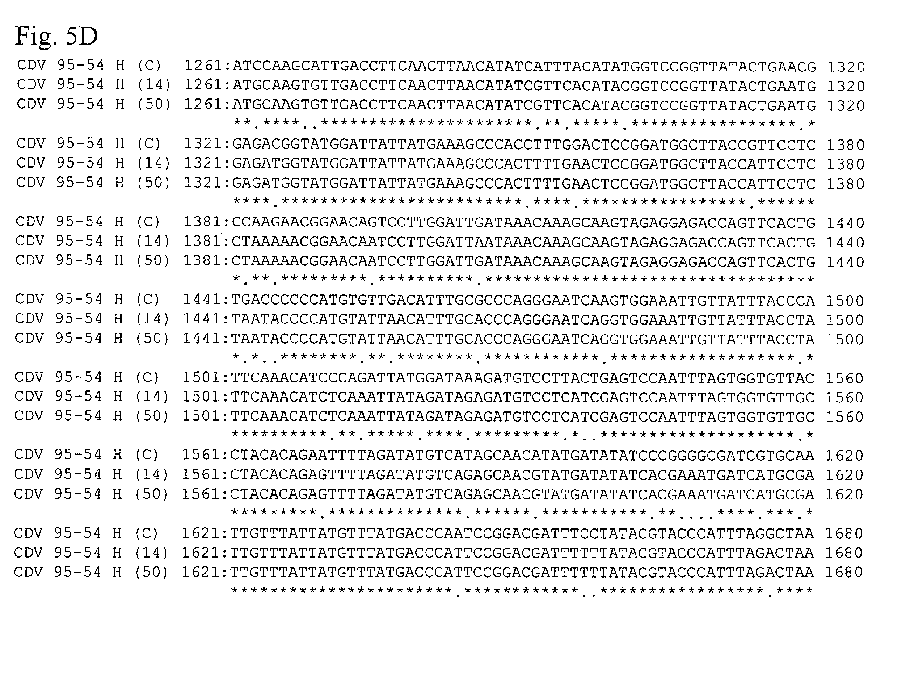

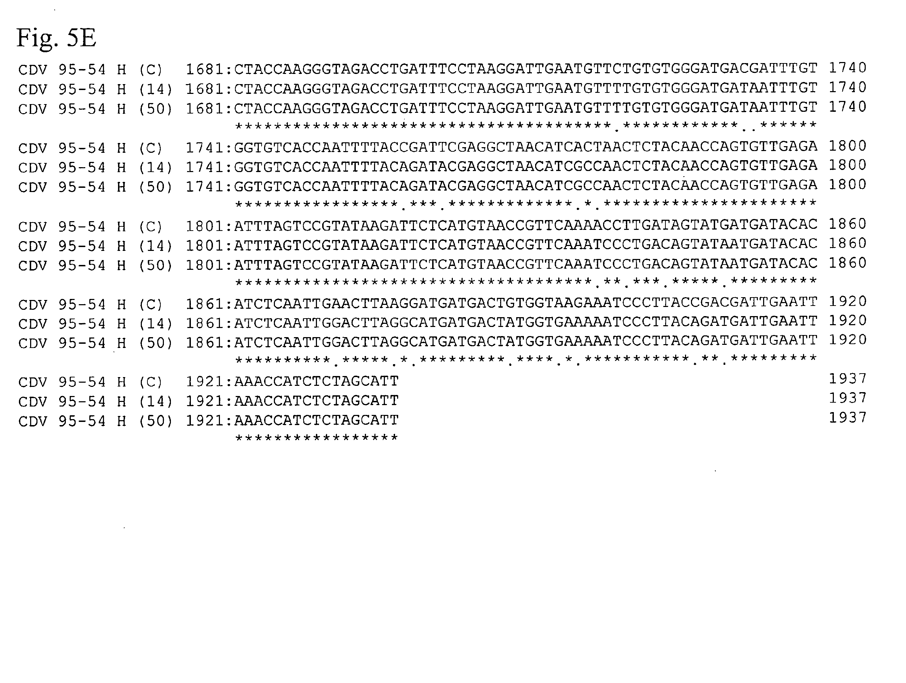

Comparison of the nucleotide sequence of the parent strain of the H gene of the canine distemper strain, the nucleotide sequence at the 14^{th} passage thereof, and the nucleotide sequence at the 50^{th} passage thereof is shown in Figs. 5A to 5E.

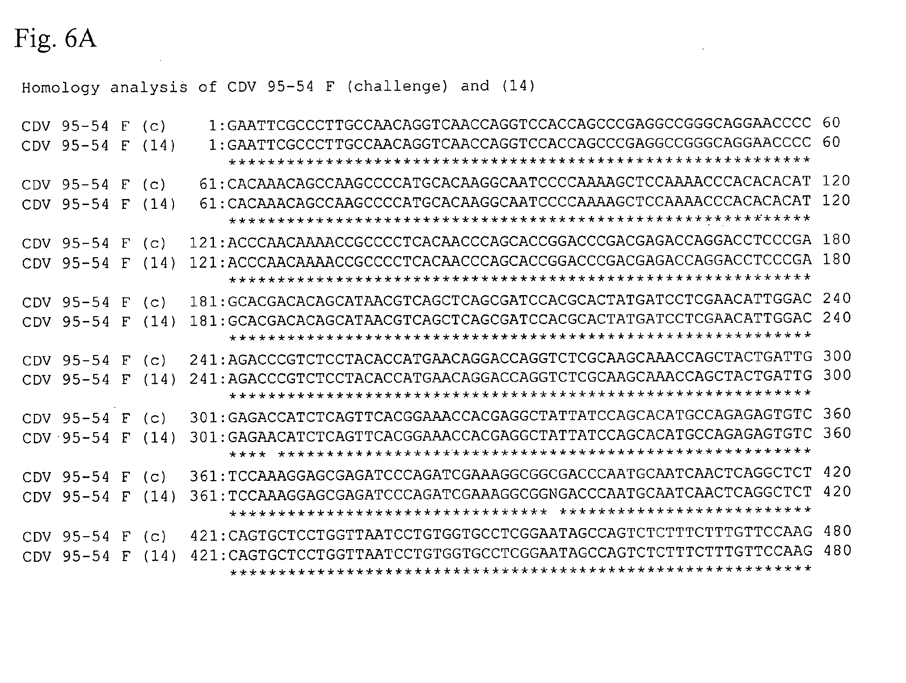

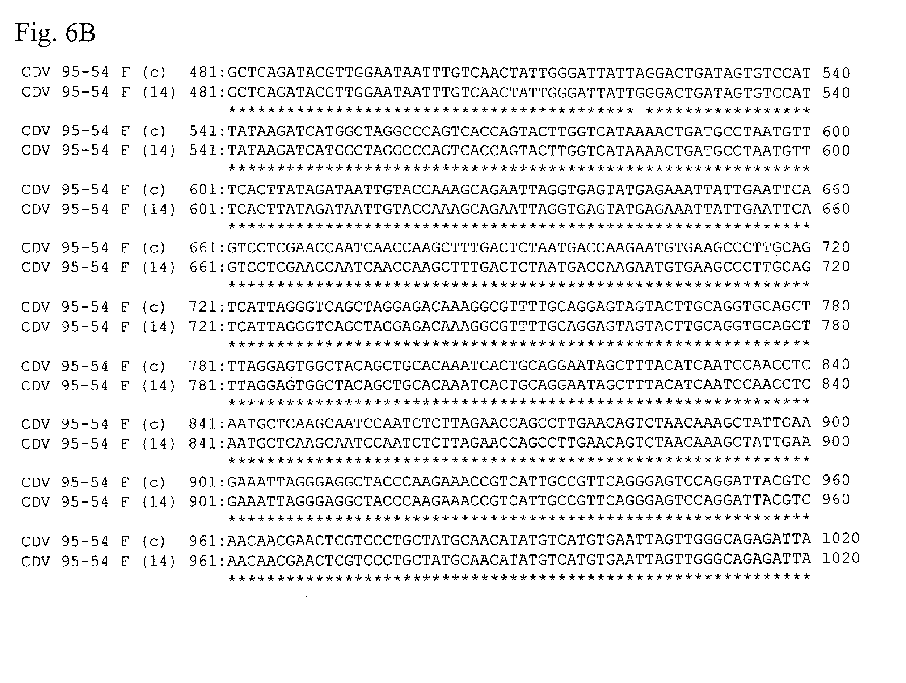

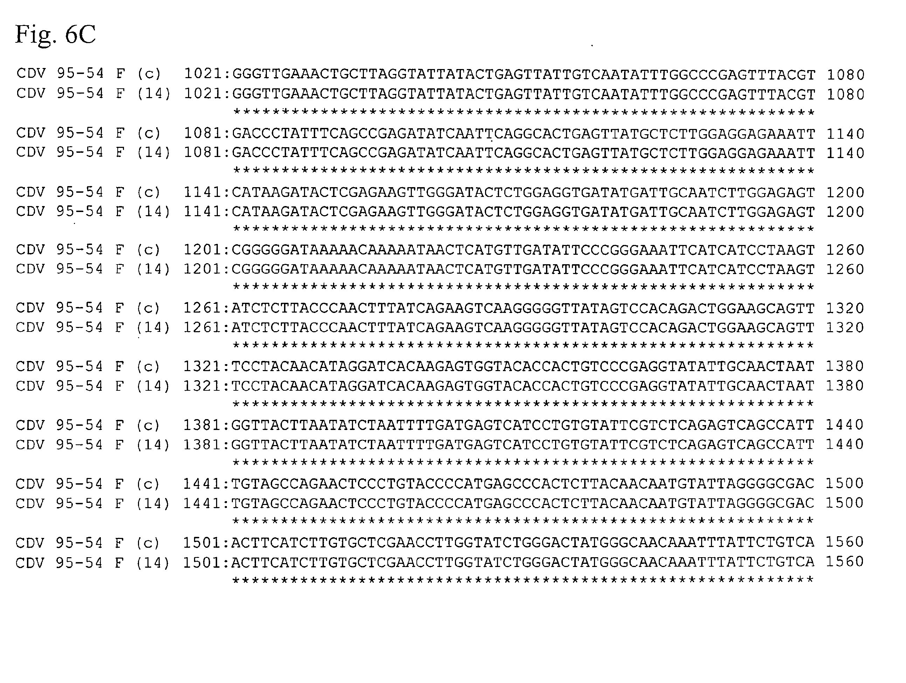

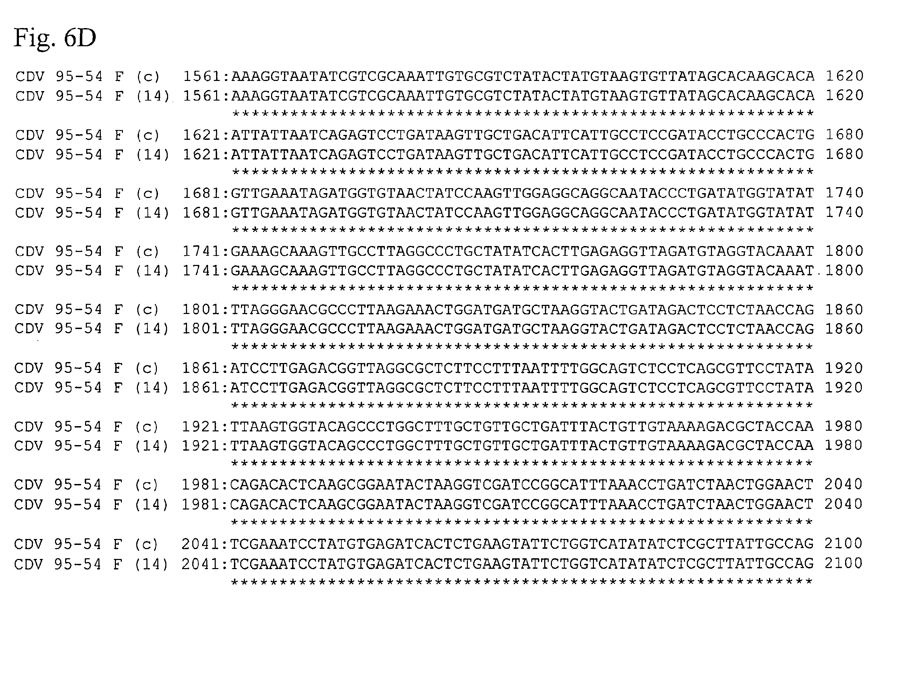

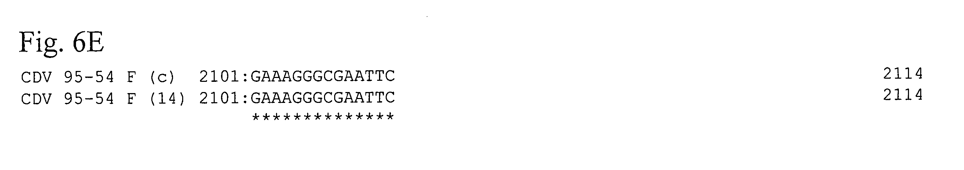

Comparison of the nucleotide sequence of the parent strain of the F gene of the canine distemper strain and the nucleotide sequence at the 14^{th} passage is shown in Figs. 6A to 6E.

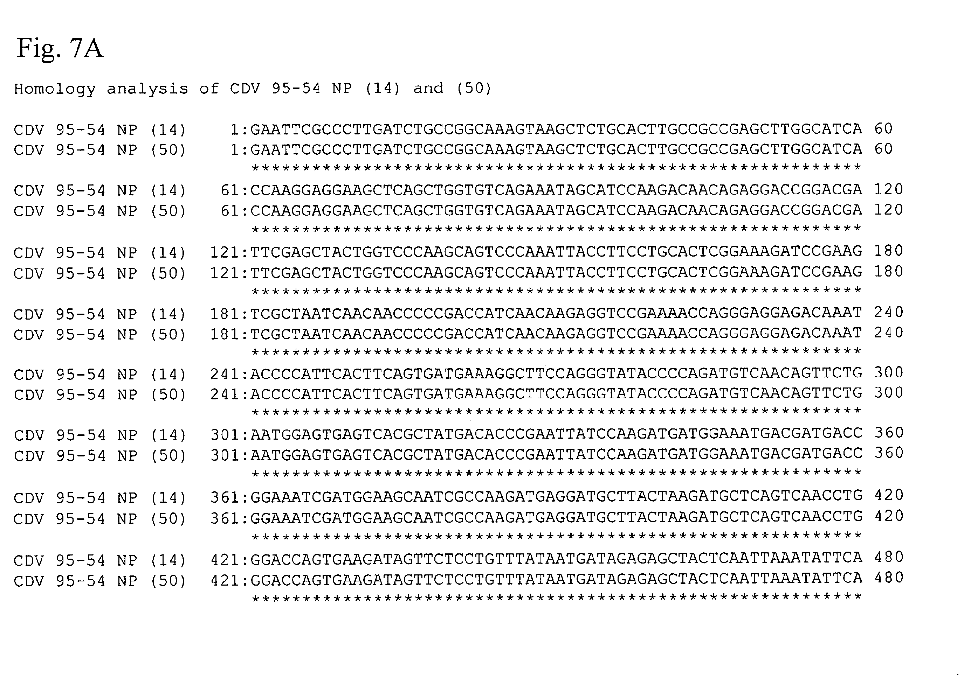

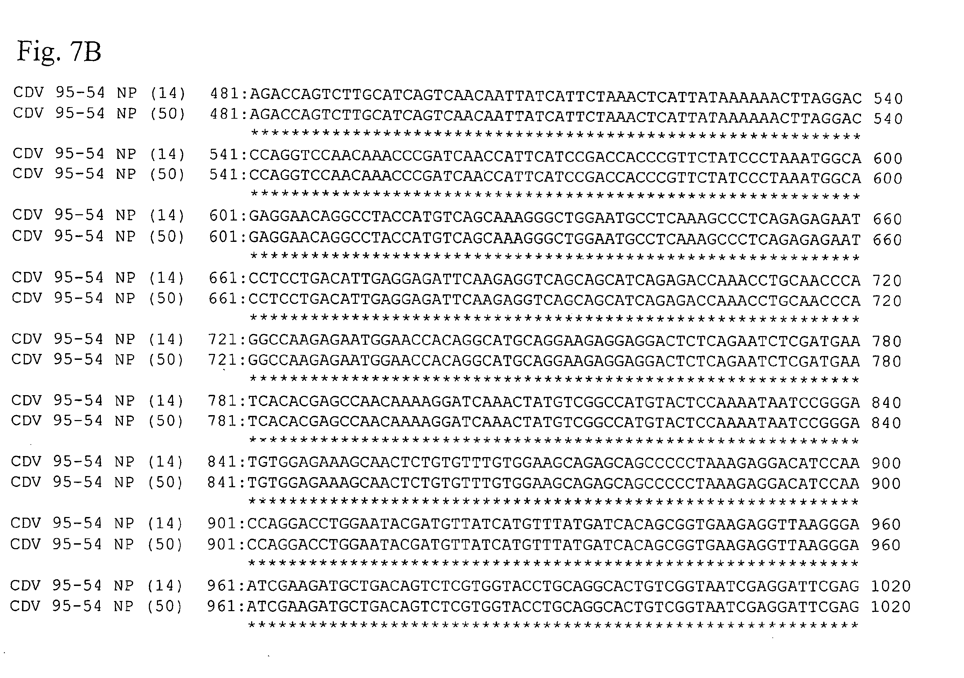

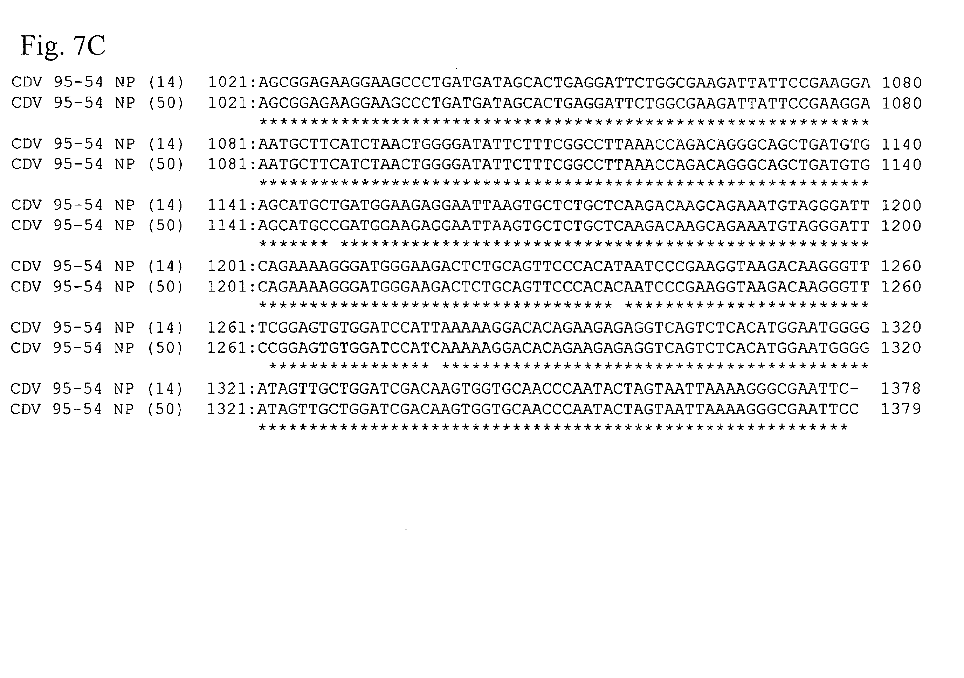

Comparison of the nucleotide sequence of the strain obtained from the NP gene of the canine distemper virus at the 14^{th} passage thereof and the nucleotide sequence of the strain at the 50^{th} passage there of is shown in Figs. 7A to 7C.

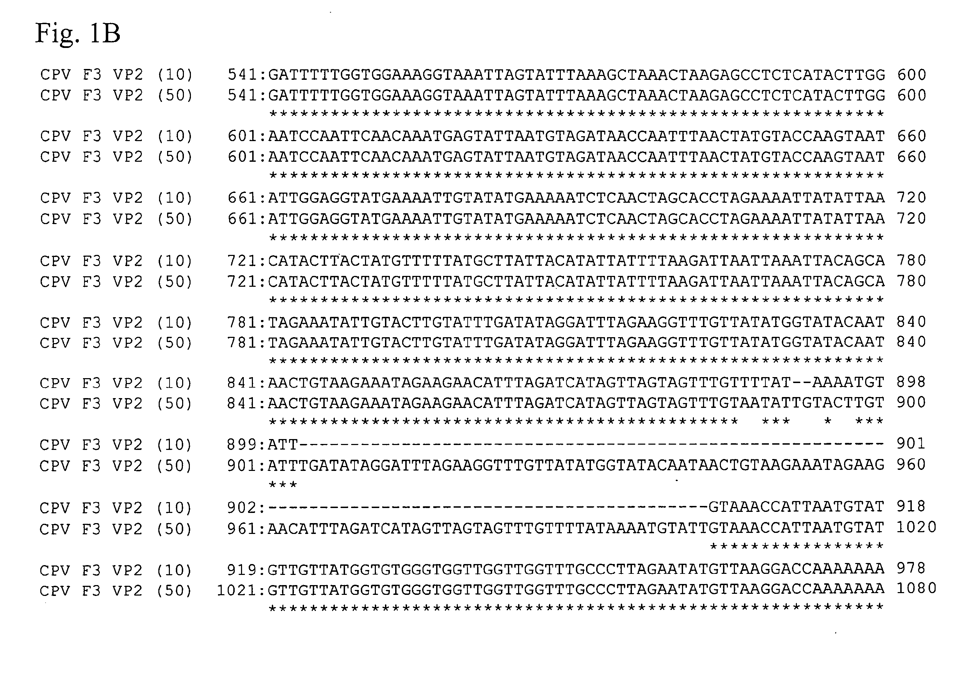

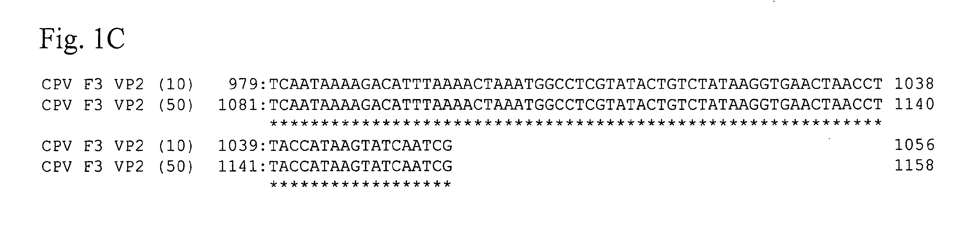

The alignments of the nucleotide sequences shown in Figs. 1 to 7 demonstrate the presence of genes that are mutated via subculture. Such gene mutations are considered to be associated with attenuation of viruses.

Nucleotides 885, 886, 892, 894, and 895 of the nucleotide sequence of the canine parvovirus VP2 gene at the 10^{th} passage have been substituted compared to the nucleotide sequence at the 10^{th} passage therof. To the nucleotide sequence of the strain at the 50^{th} passage thereof, nucleotides 892 and 893 (i.e., the region between nucleotides 891 and 892 of the nucleotide sequence at the 10^{th} passage) and nucleotides 902 to 1,003 (i.e., the region between nucleotides 901 and 902 of the nucleotide sequence at the 10^{th} passage thereof) are inserted.

Nucleotides 394 and 523 of the F gene of the parent canine distemper virus strain have been substituted in a strain at the 14^{th} passage thereof. Also, nucleotides 56, 74, 78, 94, 100, 110, 116, 128, 135, 142, 156, 159, 174, 176, 182, 204, 218, 278, 306, 311, 320, 350, 374, 389, 398, 407, 416, 437, 446, 452, 456, 482, 485, 489, 495, 499, 531, 542, 548, 551, 553, 557, 558, 572, 598, 610, 614, 626, 629, 632, 647, 650, 659, 664, 680, 689, 698, 707, 716, 732, 737, 741, 753, 755, 776, 785, 788, 792, 812, 821, 850, 872, 884, 888, 914, 923, 929, 932, 935, 946, 947, 955, 957, 968, 977, 978, 983, 999, 1010, 1011, 1016, 1019, 1044, 1051, 1067, 1076, 1079, 1094, 1096, 1115, 1116, 1120, 1136, 1139, 1146, 1147, 1160, 1176, 1184, 1214, 1232, 1255, 1263, 1268, 1269, 1292, 1295, 1301, 1319, 1325, 1351, 1356, 1374, 1382, 1385, 1395, 1406, 1442, 1444, 1445, 1454, 1457, 1466, 1479, 1499, 1511, 1514, 1520, 1525, 1535, 1537, 1538, 1559, 1570, 1585, 1592, 1604, 1607, 1608, 1609, 1610, 1615, 1619, 1644, 1657, 1658, 1676, 1720, 1733, 1734, 1758, 1762, 1776, 1778, 1838, 1840, 1845, 1851, 1871, 1877, 1879, 1889, 1894, 1896, 1908, and 1911 of the canine distemper virus H gene sequence of the parent strain have been substituted in a strain at the 50^{th} passage thereof. Further, nucleotides 628, 634, 644, 648, 657, 681, 683, 718, 744, 748, 823, and 1406 of the strain at the 14^{th} passage thereof have been substituted in a strain at the 50^{th} passage thereof.

The present invention includes the canine parvovirus F3 strain, the canine adenovirus type 2 F1 strain, and the canine distemper virus 95-54 strains, which are parent strains of the viruses isolated from a dog.

The canine adenovirus type 2 F1 strain and the canine distemper virus 95-54 strains had been deposited at European Collection of Cell Cultures (ECACC), Centre for Emergency Preparedness and Response, the Health Protection Agency, Porton Down, Salisbury, SP4 0JG, UK as of July 16, 2007 under Accession Number 07071601 and Accession Number 07071602 under the Budapest Treaty.

The canine parvovirus F3 strain has a VP2 gene consisting of the nucleotide sequence as shown in SEQ ID NO: 1 or a nucleotide sequence derived from the nucleotide sequence as shown in SEQ ID NO: 1 by deletion, substitution, or addition of one or several nucleotides and/or an N gene consisting of the nucleotide sequence as shown in SEQ ID NO: 3 or a nucleotide sequence derived from the nucleotide sequence as shown in SEQ ID NO: 3 by deletion, substitution, or addition of one or several nucleotides. The term "several" in the term "one or several" refers to not more than 9, preferably not more than 5, and further preferably 2.

The canine adenovirus type 2 F1 strain has an LITR E1A2 gene consisting of the nucleotide sequence as shown in SEQ ID NO: 5 or a nucleotide sequence derived from the nucleotide sequence as shown in SEQ ID NO: 5 by deletion, substitution, or addition of one or several nucleotides and/or an RITR gene consisting of the nucleotide sequence as shown in SEQ ID NO: 7 or a nucleotide sequence derived from the nucleotide sequence as shown in SEQ ID NO: 7 by deletion, substitution, or addition of one or several nucleotides. The term "several" in the term "one or several" refers to not more than 9, preferably not more than 5, and further preferably 2.

The canine distemper virus 95-54 strain has an H gene consisting of the nucleotide sequence as shown in SEQ ID NO: 9 or a nucleotide sequence derived from the nucleotide sequence as shown in SEQ ID NO: 9 by deletion, substitution, or addition of one or several nucleotides and/or an F gene consisting of the nucleotide sequence as shown in SEQ ID NO: 12 or a nucleotide sequence derived from the nucleotide sequence as shown in SEQ ID NO: 12 by deletion, substitution, or addition of one or several nucleotides. Further, such strain may comprise an NP gene consisting of the nucleotide sequence as shown in SEQ ID NO: 14 or a nucleotide sequence derived from the nucleotide sequence as shown in SEQ ID NO: 14 by deletion, substitution, or addition of one or several nucleotides. The term "several" in the term "one or several" refers to not more than 9, preferably not more than 5, and further preferably 2.

SEQ ID NO: 26 shows a partial sequence similar to the full-length sequence of the canine parvovirus F3 strain. The length of such sequence is approximately 75% to 80% of the full-length sequence. This sequence was analyzed via shotgun sequencing, and some low-quality nucleotides were found to be present. The canine parvovirus F3 strain of the present invention is identified as any of the following viruses:
(1) a virus having a genome comprising DNA containing the nucleotide sequence as shown in SEQ ID NO: 26;
(2) a virus having a genome comprising DNA containing a nucleotide sequence consisting of 500, 750, 1000, 1250, 1500, 1750, 2000, 2250, 2500, 2750, 3000, 3250, 3500, 3750, or 4000 continuous nucleotides of the nucleotide sequence as shown in SEQ ID NO: 26;
(3) a virus having a genome comprising DNA containing a nucleotide sequence consisting of nucleotides 188, 200, or 630 to 4000 or 4017 of the nucleotide sequence as shown in SEQ ID NO: 26;
(4) a virus having a genome comprising DNA containing a nucleotide sequence consisting of nucleotides from 188, 200, or 630 to 480 of the nucleotide sequence as shown in SEQ ID NO: 26;
(5) a virus having a genome comprising DNA containing a nucleotide sequence consisting of nucleotides from 500 to 4000 or 4017 of the nucleotide sequence as shown in SEQ ID NO: 26;
(6) a virus having a genome comprising DNA that hybridizes under stringent hybridization conditions to DNA comprising a sequence complementary to DNA consisting of a nucleotide sequence of the genome according to any of (1) to (5) above (the term "stringent conditions" used herein refers to conditions in which hybridization is carried out using a filter on which DNA has been immobilized in the presence of 0.7 to 1.0M NaCl at 68°C, following which the filter is washed with a 0.1x to 2x SSC solution (1x SSC consists of 150 mM NaCl and 15 mM sodium citrate) at 68°C to thereby identify hybrids of interest or conditions in which DNA is transferred and immobilized on a nitrocellulose membrane via Southern blotting, and in which the reaction is allowed to proceed in a hybridization buffer (50% formamide, 4× SSC, 50 mM HEPES (pH 7.0), 10× Denhardt's solution, 100 µg/ml of salmon sperm DNA) at 42°C overnight to thereby form hybrids); and
(7) a virus having a genome consisting of DNA having at least 85%, preferably 90% or higher, further preferably 95% or higher, further preferably 97% or higher, further preferably 98% or higher, and particularly preferably 99% or higher homology to DNA consisting of the nucleotide sequence of the genome according to any of (1) to (5) above, when calculated using, for example, BLAST (Basic Local Alignment Search Tool) at the
National Center for Biological Information (with the use of, for example, default i.e., initial parameters).

The attenuated virus strain of the present invention has a nucleotide sequence having mutations at the aforementioned sites of the parent virus strain.

Specifically, the attenuated virus strain of the present invention is an attenuated canine parvovirus strain obtained by adapting the canine parvovirus F3 strain in cultured cells to attenuate the same, which has a nucleotide sequence derived from the nucleotide sequence of the VP2 gene as shown in SEQ ID NO: 1 by mutation of one or a plurality of, and preferably all of, nucleotides 885, 886, 892, 894, and 895. Alternatively, the strain may comprise a nucleotide sequence comprising two nucleotides inserted into a site between nucleotides 891 and 892 and/or 100 nucleotides inserted into a site between nucleotides 901 and 902.

An attenuated canine distemper virus strain obtained by attenuating the canine distemper virus 95-54 strain via adaptation to the cultured cell which has at least 1, preferably 2, and further preferably 3 of the mutations (1) to (3) below:
(1) mutation of nucleotides 394 and/or 523 of the F gene as shown in SEQ ID NO: 12;
(2) mutation of one or a plurality of, and preferably all of, nucleotides 56, 74, 78, 94, 100, 110, 116, 128, 135, 142, 156, 159, 174, 176, 182, 204, 218, 278, 306, 311, 320, 350, 374, 389, 398, 407, 416, 437, 446, 452, 456, 482, 485, 489, 495, 499, 531, 542, 548, 551, 553, 557, 558, 572, 598, 610, 614, 626, 629, 632, 647, 650, 659, 664, 680, 689, 698, 707, 716, 732, 737, 741, 753, 755, 776, 785, 788, 792, 812, 821, 850, 872, 884, 888, 914, 923, 929, 932, 935, 946, 947, 955, 957, 968, 977, 978, 983, 999, 1010, 1011, 1016, 1019, 1044, 1051, 1067, 1076, 1079, 1094, 1096, 1115, 1116, 1120, 1136, 1139, 1146, 1147, 1160, 1176, 1184, 1214, 1232, 1255, 1263, 1268, 1269, 1292, 1295, 1301, 1319, 1325, 1351, 1356, 1374, 1382, 1385, 1395, 1406, 1442, 1444, 1445, 1454, 1457, 1466, 1479, 1499, 1511, 1514, 1520, 1525, 1535, 1537, 1538, 1559, 1570, 1585, 1592, 1604, 1607, 1608, 1609, 1610, 1615, 1619, 1644, 1657, 1658, 1676, 1720, 1733, 1734, 1758, 1762, 1776, 1778, 1838, 1840, 1845, 1851, 1871, 1877, 1879, 1889, 1894, 1896, 1908, and 1911 of the H gene as shown in SEQ ID NO: 10; and
(3) mutation of one or a plurality of, and preferably all of, nucleotides 628, 634, 644, 648, 657, 681, 683, 718, 744, 748, 823, and 1406 of the H gene as shown in SEQ ID NO: 10.

The vaccines of the present invention can be independently used as plain vaccines, such as vaccines against canine distemper, vaccines against canine adenovirus type 1 and type 2 infections, or vaccines against canine parvovirus infections, combination vaccines consisting of two separate vaccines, such as a vaccine against canine distemper and a vaccine against canine adenovirus type 1 and type 2 infections, a vaccine against canine distemper and a vaccine against canine parvovirus infections, or a vaccine against canine adenovirus type 1 and type 2 infections and a vaccine against canine parvovirus infections, or combination vaccines consisting of three separate vaccines, such as a vaccine against canine distemper, a vaccine against canine adenovirus type 2 infection, and a vaccine against canine parvovirus infections.

The vaccines of the present invention would not cause interference among viruses even if the vaccines are administered in combination, and the antibody titers would be elevated regarding all the combined attenuated virus strains. In particular, use of combination vaccines containing vaccines against canine distemper virus and vaccines against canine parvovirus would not cause interference.

The doses of the vaccines of the present invention are the same as the doses of known vaccines. The canine distemper virus content in the vaccines is about 1 × 10² to about 1×10¹² 50% tissue culture infective dose (TCID₅₀), the canine adenovirus type 2 virus content therein is about 1×10² to about 1×10¹² 50% tissue culture infective dose (TCID₅₀), and the canine parvovirus content therein is about 1×10² to about 1×10¹² 50% tissue culture infective dose (TCID₅₀). TCID₅₀ values of the vaccines are measured in accordance with development of cytopathic effects (CPE). The term "CPE" refers to phenomena where cultured cells exhibit various configurations, such as granular, circular, or swollen configurations and finally drop out of the culture vessel to float in a maintenance solution. CPE can be confirmed under a microscope.

The vaccines of the present invention comprise, as active ingredients, effective amounts of the virus vaccines of the present invention from a veterinary point of view. The vaccines may be in the form of an aqueous or non-aqueous sterilized solution, a suspension, or an emulsion. Further, the vaccines may comprise a pharmaceutically acceptable diluent, such as salt, buffer, or adjuvant, an auxiliary agent, or a carrier. The vaccines can be administered through various routes, such as oral, nasal, transmucosal, intramuscular, subcutaneous, intranasal, intratracheal, cutaneous, percutaneous, or intradermal routes. Oral administration, nasal administration, and transmucosal administration are particularly preferable. The vaccines of the present invention may be incorporated into drinking water or feeds and fed to a dog. The present invention includes drinking water and feeds containing the vaccines.

The vaccines of the present invention comprise maternal antibodies obtained from mother dogs and thus can immunize puppies, which cannot be immunized via administration via conventional injection administration due to elimination of vaccine viruses by maternal antibodies. Target puppies are 4- to 18-week-old, and preferably 4- to 8-week-old puppies who have received maternal antibodies.

### Examples

The present invention is described in detail with reference to the following examples, whereas the technical scope of the present invention is not limited thereto.

### Example 1: Attenuation of virus

The parent virus strain was isolated in the following method.

The canine parvovirus F3 strain was isolated from stool of a mixed-breed dog obtained from the public health center of Kooriyama, Fukushima, in accordance with a conventional technique.

The canine adenovirus type 2 F1 strain was isolated from a throat smear or the like of an in-house-bred beagle dog raised in Nippon Zenyaku Kogyo Co., Ltd. in accordance with a conventional technique.

The canine distermper virus strain isolated by Nippon Zenyaku Kogyo Co., Ltd from a dog nasal specimen received when externally assigned to conducting the analysis.

Viruses were subcultured in the following manner.

### In the case of canine distemper virus and canine adenovirus type 2 virus strains

1. Cells are subcultured.
2. Medium is removed upon formation of a monolayer.
3. A virus solution is innoculated
4. Incubation at 37°C for 1 hour to allow attachment of the virus to cells
5. Cell maintenance medium is added.
6. Cells are incubated in a CO₂ incubator at 37°C.
7. When CPE is observed, cells are frozen at -80°C while part of them are subjected to further subculture.

### In the case of canine parvovirus strains

1. Cells are subcultured.
2. A virus solution is inoculated.
3. Cells are incubated in a CO₂ incubator at 37°C.
4. When CPE is observed, cells are frozen at -80°C while part of them are subjected to further subculture.

### Example 2: Sequencing of a particular region of virus gene

### Sequencing of canine distemper virus (CDV) H and F genes

A virus solution (2 to 3 ml) was innoculated onto Vero cells, which had grown into a full-sheet formation in a 10-cm petri dish for cell culture (Falcon). Virus attachment was allowed at 37°C for 1 hour before 15 ml of growth medium was added, and culture was incubated at 37°C for 3 to 4 days. When the cytopathic effects reached about 50% of the whole, total RNA was extracted from the virus-infected cells using a commercially available RNA extraction kit (TRIZOL reagent, Life Technologies Oriental., Inc.). Procedures were in accordance with the attached instructions. Specifically, medium was removed from the petri dish, 1 ml of TRIZOL reagent was added to the cells, cells were homogenized via pipetting, the resulting cell lysate was transferred to a 1.5-ml centrifugation tube, and the resultant was kept at room temperature for 5 minutes. Chloroform (0.2 ml) was added and mixed therewith, and the resultant was kept at room temperature for 3 minutes. After centrifugation (at 12,000× g for 15 minutes at 4°C), the aqueous layer was transferred to the other centrifugation tube and then subjected to isopropanol precipitation. The pellet was washed via centrifugation with 75% ethanol (at 7,500× g for 5 minutes at 4°C), air-dried, and dissolved in 0.5 to 1.0 µg/µl of diethyl pyrocarbonate (DEPC)-treated water. The resultant was designated as a virus RNA solution.

Reverse transcription was carried out using a 0.5-ml tube, 1 µl of a virus RNA solution, 0.25 µl of a CDV H or F reverse primer, 2 µl of 5× 1st Strand Buffer (included with the reverse transcriptase), and 4.55 µl of DEPC water were added to bring the total amount of the solution to 7.8 µl, and a drop of mineral oil (Sigma, free of DNase, RNase, and protease) was superposed thereon. The resultant was heated at 70°C for 10 minutes and then ice-cooled immediately thereafter. Subsequently, 0.5 µl of M-MLV reverse transcriptase (200 units/µl, Life Technologies Oriental., Inc.), 0.5 µl of deoxynucleotide mixture (Pharmacia), 1 µl of 0.1M dithiothreitol, and 0.2 µl of RNase inhibitor (RNasin; 40 units/µl, TOYOBO) were added to prepare 10 µl of RT reaction solution in total, and the reaction was allowed to proceed at 37°C for 1 hour to synthesize cDNA of mRNA of the CDV H and F genes. After the completion of the reaction, the product was heated at 70°C for 10 minutes.

To the total amount of the RT reaction solution, 10 µl of 10× PCR Buffer (included with Taq DNA polymerase), 0.5 µl each of primers (CDV H forward and reverse primers or CDV F forward and reverse primers), 8 µl of dNTPs Mixture (2.5 mM each, included with Taq DNA polymerase), 70.5 µl of sterile distilled water, and 0.5 µl of Taq DNA polymerase (TaKaRa EX Taq; 5 units/µl, Takara Shuzo Co., Ltd.) were added to prepare 100 µl of PCR reaction solution in total. As a DNA amplifier used for PCR, a Thermal Cyclic Reactor (TC-100, Toyobo Engineering Co., Ltd.) was used, the PCR reaction solution was applied thereon, and a cycle of 94°C for 1 minute, 58°C for 1 minute, and 72°C for 2 minutes was repeated 30 times. After the completion of the reaction, part of the reaction solution was collected and subjected to electrophoresis on 0.7% agarose gel, stained with ethidium bromide, and irradiated with ultraviolet rays to confirm amplification of DNA of the CDV H and F genes.

Thereafter, sequencing and gene analysis were assigned to Gene Analysis Center, Takara Shuzo Co., Ltd.

### Sequencing of canine adenovirus type 2 (CAV2) gene

MDCK cells, which had grown into a full-sheet formation in a 75 cm² rectangular culture flask, were washed with 10 ml of phosphate buffer and 1 ml of virus solution was innoculated therein. The resultant was kept at 37°C for 30 minutes to adsorb the viruses, 20 ml of cell growth medium was added thereto, and stationary culture was carried out at 37°C for 40 hours.

Virus DNA was extracted from virus-infected cells in accordance with the method of Shinagawa et al. (Shinagawa et al., Microbiol. Immunol., 27, 817-822, 1983).

Culture medium was removed, 2 ml of cell lysate was added, the mixture was kept at room temperature for 10 minutes, and the resultant was incubated at 37°C for 5 minutes. 5M sodium chloride (0.5 ml) was added thereto, the mixture was subjected to mild reverse agitation, and the resultant was incubated at 37°C for 5 minutes, followed by ice cooling for 3 to 4 hours. Centrifugation was carried out at 10,000× g for 5 minutes; 10 mM Tris-HCl buffer-saturated phenol, was added to the equivalent amount of the supernatant; and the mixture was agitated at room temperature for 10 minutes, followed by centrifugation at 16,000× g for 10 minutes. The aqueous layer was removed, and 10 mM Tris-HCl buffer was added to the equivalent amount of the phenol layer, followed by agitation and centrifugation using the same method. The aqueous layer was completely removed, 1.5x volume of ethanol was added to the phenol layer, and the resultant was ice cooled for 10 minutes, followed by centrifugation at 4,000× g for 10 minutes.

The pellet was washed with 0.5 ml of 80% ethanol, and was dissolved in 0.5 ml of TENS solution, 250 to 500 µg of Proteinase K powder was added thereto, and the mixture was incubated at 37°C for about 1 hour until insoluble matter became unobservable. Phenol: chloroform (1:1) was added to the equivalent amount of the enzymolysis solution, the mixture was subjected to the same procedure. Chloroform was added to the equivalent amount of the aqueous layer, and the resultant was treated using the same method. To the resulting aqueous layer, 1/10 volume of 3M sodium acetate solution and 1 ml of ethanol were added, and the resultant was kept at -70°C for 1 hour, followed by centrifugation at 10,000× g for 10 minutes. The pellet was washed with 80% ethanol and dissolved in 20 µl of TE buffer to prepare a virus DNA solution.

Thereafter, sequencing and gene analysis were assigned to the Gene Analysis Center, Takara Shuzo Co., Ltd.

### Sequencing of canine parvovirus (CPV) VP-2 gene

The CPV VP-2 gene was analyzed in the following method. 2 to 3 ml of virus solution was added to 5 ml of the A72 cell suspension (7×10⁵/ ml) prepared in a growth medium, and the resultant was plated in a 25-cm² rectangular culture flask (Falconand incubated at 37°C for 3 to 4 days. The culture medium was recovered when the cytopathic effects were evenly spread. After centrifugation (3,000 rpm, 10 minutes), the supernatant was diluted 100-fold with sterile distilled water, and the resultant was designated as a virus diluent.

5 µl of virus diluent, 10 µl of 10× PCR Buffer (included with the Taq DNA polymerase), 0.5 µl each of the primers (VP-2 forward and reverse primers), 8 µl of dNTPs Mixture (included with Taq DNA polymerase), and sterile distilled water were added to a 0.5-ml tube to bring the amount of the solution to 95 µl. A drop of mineral oil (Sigma, free of DNase, RNase, and protease) was superposed thereon and the resultant was applied to a DNA amplifier (Thermal Cyclic Reactor, TC-100, Toyobo Engineering Co., Ltd.), followed by heating at 94°C for 5 minutes. Subsequently, 0.5 µl of Taq DNA polymerase (TaKaRa Ex Taq; 5 units/µl, Takara Shuzo Co., Ltd.) and sterile distilled water were added to prepare 100 µl of PCR reaction solution in total, and a cycle of 94°C for 30 seconds, 55°C for 2 minutes, and 72°C for 2 minutes was repeated 30 times. After the completion of the reaction, part of the reaction solution was collected and subjected to electrophoresis on 0.7% agarose gel, stained with ethidium bromide, and irradiated with ultraviolet rays to confirm amplification of DNA of the genes.

Thereafter, sequencing and gene analysis were assigned to the Gene Analysis Center, Takara Shuzo Co., Ltd.

In sequencing, RT-PCR was carried out using the following primers.
CDV H forward primer: 5'-AATGCTAGAGATGGTTTAATT-3' (SEQ ID NO: 16)
CDV H reverse primer: 5'-AGGGCTCAGGTAGTCCAGC-3' (SEQ ID NO: 17)
CDV F forward primer: 5'-CATAGCAAGCCAACAGGTCAACCAGG-3' (SEQ ID NO: 18)
CDV F reverse primer: 5'-CCTGGCAATCAAGCGAGATATATG-3 (SEQ ID NO: 19)
CPV VP-2 forward primer: 5'-ATGAGTGATGGAGCAGTTCAACCA-3' (SEQ ID NO: 20)
CPV VP-2 reverse primer: 5'-ATATAATTTTCTAGGTGCTAGTTGAG-3' (SEQ ID NO: 21)

### Example 3: Efficacy evaluation consisting of immunogenic test and protection test and safety assessment consisting of pathogenic reversion test regarding vaccines against canine distemper virus

### (1) Efficacy evaluation

In order to investigate whether or not the canine distemper virus (CDV) 95-54 strains possess high immunogenicity via oral administration, the strains attenuated via subculture for 24 passages in Vero cells were administered to dogs, and production of neutralizing antibodies against CDV was compared.

Twelve roughly 3-month-old beagle dogs were divided into three groups: i.e., a group of 5 dogs subjected to oral administration, a group of 2 dogs subjected to subcutaneous injection, and a untreated group of 5 dogs in accordance with their sex and age in months. To the test dogs of the group subjected to oral administration, 2 ml of culture supernatants of 10^{5.5}/ml CDV 95-54 strains were administered per dog twice orally and nasally (1 ml each) at intervals of 3 weeks. Highly virulent strains were challenged nasally and orally by fixing a 19 G injection needle on the acral part of a nozzle of a spray catheter for a fiber scope (PW-6C-1, Olympus Corporation), which had been cut to about 10 cm. Catheter was inserted to the right nasal cavity of a CDV-antibody-negative dog, and the spray was conducted in the nasopharynx. To the test dogs of the group subjected to subcutaneous injection, 2 ml of culture supernatants of 10^{5.5}/ml CDV 95-54 strains were subcutaneously injected twice per dog at intervals of 3 weeks. The test dogs were subjected to general clinical observation every day from 2 days before the first administration to 3 weeks after the second administration. Blood samples were collected from both groups every week from the day of the first administration to the completion of the test, and CDV antibody titers were measured.

Also, whether or not the immunized dogs could be protected from infection via nasal or oral challenge was investigated using intact highly virulent CDV95-54 as challenge viruses for protection. Highly virulent strains were challenged by nasally or orally administering 1 ml of culture supernatant of a highly virulent 10^{5.5}/ml CDV95-54 strain once in the same method as with the method for immunogenicity confirmation. The blood serum samples were collected every week from before virus inoculation to 21 days after inoculation, and CDV neutralizing antibody titers were measured in accordance with a conventional technique.

As shown in Table 1, satisfactory immunogenicity as vaccines and effects of protection were observed in both the group subjected to subcutaneous administration and the group subjected to nasal or oral administration.

**Table 1**

| Transition in CDV-neutralizing antibody titer | | | | | | | | | | | | |
|---|---|---|---|---|---|---|---|---|---|---|---|---|
| Group | Dog number | Antibody production confirmation test | | | | | | | Protection test | | | |
| | | 0W(1st) | 1W | 2W | 3W(2nd) | 4W | 5W | 6W | 0d(Challenge) | 7d | 14d | 21d |
| Group subjected to subcutaneous injection | 03-79 | <4 | 8 | 405 | 512 | 6,472 | 2,048 | 1,024 | 648 | 648 | 405 | 512 |
| | 03-81 | <4 | 16 | 88 | 256 | 4,096 | 1,024 | 744 | 512 | 512 | 256 | 405 |
| | Geometric average | <4 | 11 | 189 | 362 | 5,149 | 1,448 | 873 | 576 | 576 | 321 | 455 |
| Group subjected to nasal or oral administration | 04-01 | <4 | 3 | 405 | 2,048 | 2,048 | 1,618 | 1,024 | 1,618 | 1,024 | 648 | 1,024 |
| | 04-04 | <4 | 10 | 405 | 1,024 | 1,024 | 648 | 512 | 512 | 512 | 512 | 512 |
| | 04-05 | <4 | 8 | 51 | 128 | 162 | 256 | 128 | 128 | 128 | 128 | 128 |
| | 04-07 | <4 | <4 | 352 | 744 | 648 | 1,024 | 1,024 | 1,618 | 648 | 2,048 | 1,024 |
| | 04-09 | <4 | 3 | 1,024 | 648 | 1,409 | 1,024 | 648 | 1,618 | 648 | 648 | 648 |
| | Geometric average | <4 | 5 | 313 | 664 | 791 | 776 | 537 | 774 | 490 | 563 | 537 |
| Untreated group | 04-02 | <4 | ND | ND | ND | ND | ND | <4 | <4 | <4 | <4 | 4 |
| | 04-03 | <4 | ND | ND | ND | ND | ND | <4 | <4 | <4 | <4 | <4 |
| | 04-06 | <4 | ND | ND | ND | ND | ND | <4 | <4 | <4 | <4 | <4 |
| | 04-08 | <4 | ND | ND | ND | ND | ND | <4 | <4 | <4 | 16 | 1,024 |
| | 04-10 | <4 | ND | ND | ND | ND | ND | <4 | <4 | <4 | 2,592 | 6,472 |

### (2) Safety assessment

The culture supernatants of the 95-54 strains (subcultured in Vero cells for 24 passages; 10^{5.89} TCID₅₀/ml) were used as immunodeficiency viruses for the pathogenic reversion test.

Roughly 3- to 3.5-month-old healthy and CDV-antibody-negative beagle dogs were employed as the test animals.

**Table 2**

| Number of passages | Test | Number of subjects |
|---|---|---|
| 1 passage | Pathogenic test and pathogenic reversion test | 5 |
| 2 passages | Pathogenic reversion test | 2 |
| 3 passages | Pathogenic reversion test | 2 |
| 4 passages | Pathogenic reversion test | 2 |
| 5 passages | Pathogenic test and pathogenic reversion test | 5 |

The first passage and the fifth passage of the pathogenic reversion test were subjected to the pathogenic test. Specifically, 1 ml of each of the culture supernatants of the 95-94 strains, which had been subcultured in Vero cells for 24 passages, was applied dropwise to the nasal cavities and the oral cavities of 5 test dogs using a syringe. The test period was from 4 days before innoculation (-4d) to 21 days after innoculation, and the following test was carried out.

For virus subculture of the pathogenic reversion test, viruses were detected from the blood and the stool every day and subjected to subculture in accordance with a conventional technique.

The items of the pathogenic test are as shown below.

**Table 3**

| Test items | Period | Remarks |
|---|---|---|
| General clinical observation | -2d to 14 d: every day | Vigor, appetite |
| | | Gastrointestinal symptoms (diarrhea, vomition) |
| | | Measurement of body temperature |
| | 0d, 7d, 14d: every week | Measurement of body weight |
| Hematological test | -2d to 14 d: every day | Red blood cell count |
| | | Hemoglobin value |
| | | Hematocrit value |
| | | Leukocyte count |
| | | Platelet count |
| | | Total plasma protein |
| Virus detection | 0d to 14d: every day | Detection of CPV gene from blood and stool (PCR) |
| | 14d | Detection of CPV gene from sampled organ (PCR) |
| | | Sampled organ: thymic gland, tonsilla, heart, liver, spleen |
| | | kidney, stomach, duodenum, jejunum, ileum |
| | | cecum, colon, rectum, mesenteric lymph node, |
| | | bone marrow, adrenal gland, bladder |
| Antibody titer measurement | 0d, 7d, 14d: every week | Neutralizing antibody titer |
| Pathological test | 14d | Anatomicopathological test, histopathological test |
| | | Sampled organ: thymic gland, tonsilla, heart, liver, spleen |
| | | kidney, stomach, duodenum, jejunum, ileum |
| | | cecum, colon, rectum, mesenteric lymph node, |
| | | bone marrow, adrenal gland, bladder |

As a result of the pathogenicity test, the attenuated virus strain was found to cause transient mild onset of fever in only one dog, but the strain of interest was not found to be problematic in terms of pathogenicity as a vaccine. Also, reversion of pathogenicity was not observed.

### Example 4: Efficacy evaluation consisting of immunogenic test and protection test and safety assessment consisting of pathogenic reversion test regarding vaccines against canine parvovirus

### (1) Efficacy evaluation

### CPV immunogenic test

The culture supernatants of the CPV F3 strains (10^{5.30}/ml; subcultured in Vero cells for 68 passages) were used as a test virus.

As test animals, 5 roughly 3-month-old beagle dogs were used. The culture supernatants of the CPV F3 strains were orally and nasally administered thereto (1 ml each; 2 ml/subject) twice at intervals of 3 weeks. The test dogs were subjected to general clinical observation every day from 2 days before the first administration to 3 weeks after the second administration. Blood samples were collected every week from the day of the first administration to the completion of the test, and CPV-HI values were measured for CPV antibody titer.

### CPV protection test

As the test viruses, the culture supernatants of the F3 strains (10^{5.00} TCID₅₀/ml, subcultured in Vero cells for 68 passages) were used.

As challenge viruses, the wild-type F3 highly virulent strains were used.

Roughly 4- to 5-month-old healthy and CPV-antibody-negative beagle dogs were employed as the test animals. A total of 8 dogs; i.e., a group consisting of 5 beagle dogs subjected to inoculation with vaccine candidate strains and a control group of 3 dogs, were employed.

Upon completion of the period of immunity (i.e., at week 6), 1 ml of the highly virulent F3 strain was administered orally to all the test dogs for virus challenge. The test period was from 2 days before challenge (-2d) to 14 days after challenge (14d), the blood samples were collected every day, and the presence of CPV in the blood was identified via detection of CPV DNA with PCR. Also, the test and the examination as shown in Table 3 were carried out (the antibody titer was measured in terms of HI antibody titer).

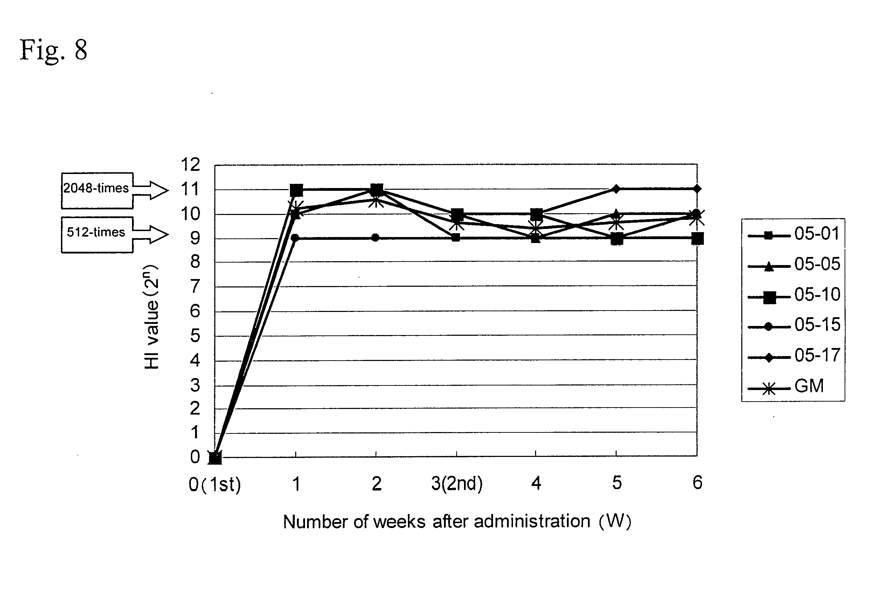

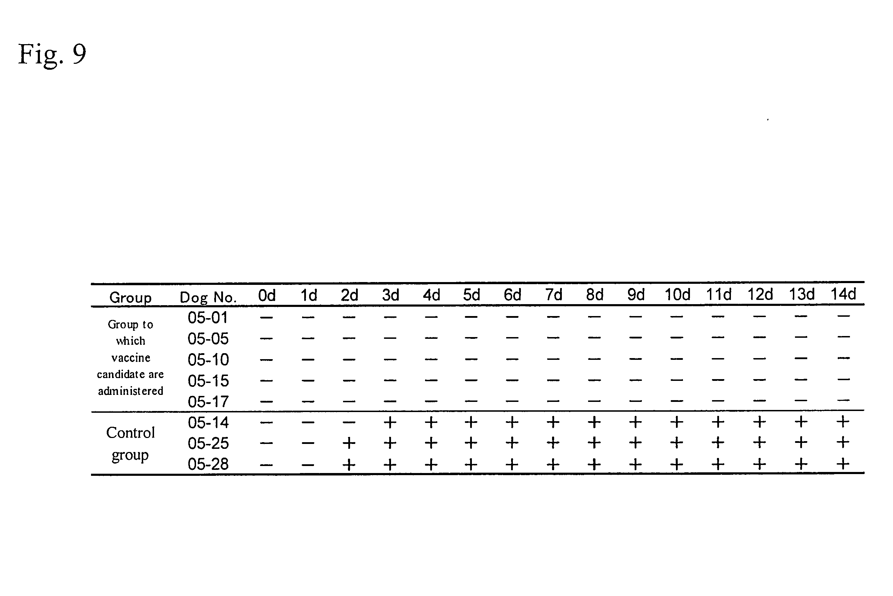

The results are shown in Fig. 8 and in Fig. 9. The attenuated parvovirus used had sufficient immunogenicity as a vaccine, and it was capable of protecting the dog from infection.

### (2) Safety assessment

### CPV pathogenic reversion test

As the test viruses, the culture supernatants of the F3 strains (10^{5.00} TCID₅₀/ml, subcultured in Vero cells for 68 passages) were used.

17 roughly 3-month-old healthy and CPV-antibody-negative beagle dogs were employed as the test animals.

The first passage and the fifth passage of the pathogenic reversion test were subjected to the pathogenic test. At the outset, 1 ml of each of the culture supernatants of the F3 strains was applied dropwise to the nasal cavities and the oral cavities of 5 test dogs using a syringe. For virus subculture of the pathogenic reversion test, viruses were detected from the blood and the stool every day and subjected to subculture in accordance with a conventional technique. The items of the pathogenic test are as described below.

The test items are as shown in Table 3, and the antibody titer was measured in terms of the HI value.

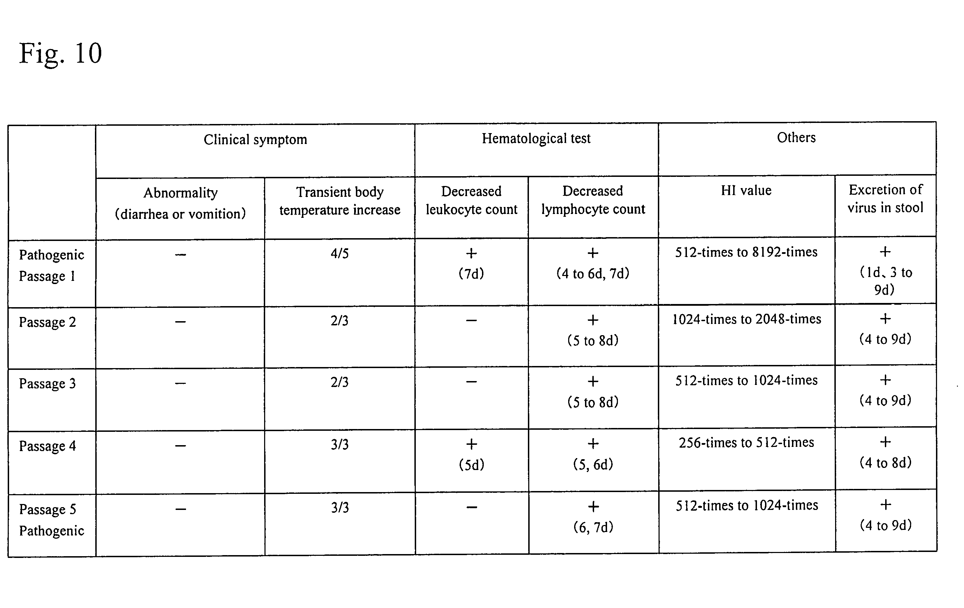

The results are shown in Fig. 10. As a result of the pathogenicity test, the attenuated virus strain was found to have no pathogenicity. Also, reversion of pathogenicity was not observed.

### Example 5: CAV2 immunogenic test

The culture supernatants of the CAV F1 strains (10⁴, 10^{5.5}, 10⁷/ml, control group, subcultured in MDCK cells for 105 passages) were used as test viruses.

As test animals, 16 roughly 3-month-old beagle dogs were used.

The culture supernatants of the CAV F1 strains were orally administered (1 ml each) to each dog of the group of 4 roughly 3-month-old subject beagle dogs. Blood samples were collected every week from the day of the first administration to the completion of the test, and CAV antibody titer was measured. The attenuated canine adenovirus type 2 vaccines were found to have immunogenicity.

### Example 6: Test of efficacy of combination vaccines

Whether or not oral administration of the above vaccine strains in combination would endow immunization was investigated. Representative vaccines listed below, which are commercially available in Japan, were used as comparative test vaccines.

As the vaccine of the present invention, a virus mixture of three types of vaccine strains; i.e., the canine distemper virus (CDV) strains attenuated via subculture in Vero cells for 24 passages, the canine adenovirus type 2 (CAV2) strains attenuated via subculture in MDCK cells for 105 passages, and the canine parvovirus (CPV) strains attenuated via subculture in A72 cells for 68 passages, was used:
1. Vanguard 5: combination live vaccines against distemper, canine adenovirus (type 2) infections, canine parainfluenza, and canine parvovirus infections (serial number: 478101; Pfizer Inc);
2. Duramune 5: combination live vaccines against distemper, canine adenovirus (type 2) infections, canine parainfluenza, and canine parvovirus infections (serial number: 101152A; Kyoritsu Seiyaku Corporation);
3. "KYOTO BIKEN" CANINE-6: combination live vaccines against distemper, canine adenovirus (type 2) infections, canine parainfluenza, canine parvovirus infections, and canine coronavirus infections (serial number: 7-2; KyotoBiken Laboratories, INC); and
4. the vaccines of the present invention: a virus mixture against distemper, canine adenovirus (type 2) infections, and canine parvovirus infections.

Groups each consisting of 3 roughly 3-month-old CDV-antibody-negative beagle dogs were randomly assigned for administration of the vaccines of the manufacturers. A dose of a vaccine (i.e., 1 ml of virus solution, 10^{5.89} TCID₅₀/ ml) was administered orally to each thereof twice at intervals of 3 weeks. The blood serum samples were collected every week from before vaccination to 6 weeks after vaccination, and CDV neutralizing antibody titers were measured in accordance with a conventional technique. Since cross antigenicity could differ depending on vaccine strain, vaccine strains isolated from relevant vaccines were used as viruses for neutralizing.

Table 1 shows CDV neutralizing antibody titers of dogs to which vaccines had been orally administered. Antibody production was observed 2 weeks after vaccination in 1 of the 3 dogs to which Vanguard had been administered, although the other 2 dogs remained antibody-negative. While antibody production was observed 5 weeks after vaccination in 1 dog to which Duramune had been administered, the antibody titer was low, and the other 2 dogs remained antibody-negative. Antibody production was observed in 1 dog at 3 weeks, another dog at 6 weeks after vaccination , to which CANINE had been administered, while a dog remained antibody-negative. The results demonstrate that oral administration of commercially available vaccines cannot completely impart immunity against CDV.

There was no significant difference among groups in transitions in antibody titers against CPV and CAV2.

**Table 4**

| Transition in CDV-neutralizing antibody titer | | | | | | | | |
|---|---|---|---|---|---|---|---|---|
| Group | Dog number | After vaccination | | | | | | |
| | | 0W (1st) | 1W | 2W | 3W (2nd) | 4W | 5W | 6W |
| Vanguard (Pfizer) | 04-18 | <4 | <4 | 128 | 512 | 2,048 | 2,048 | 1,618 |
| | 04-23 | <4 | <4 | <4 | <4 | <4 | <4 | <4 |
| | 04-32 | <4 | <4 | <4 | <4 | <4 | <4 | <4 |
| | Geometric average | <4 | <4 | 5 | 8 | 13 | 13 | 12 |
| Duramune (Kyoritsu) | 04-21 | <4 | <4 | <4 | <4 | <4 | 8 | 32 |
| | 04-31 | <4 | <4 | <4 | <4 | <4 | <4 | <4 |
| | 04-33 | <4 | <4 | <4 | <4 | <4 | <4 | <4 |
| | Geometric average | <4 | <4 | <4 | <4 | <4 | 2 | 3 |
| CANINE (KyotoBiken) | 04-20 | <4 | <4 | <4 | 128 | 162 | 405 | 512 |
| | 04-22 | <4 | <4 | <4 | <4 | <4 | <4 | <4 |
| | 04-34 | <4 | <4 | <4 | <4 | <4 | <4 | 6 |
| | Geometric average | <4 | <4 | <4 | 5 | 5 | 7 | 15 |
| Vaccine of the present invention | 05-51 | <4 | 4 | 256 | 4,096 | 2,048 | 1,024 | 648 |
| | 05-53 | <4 | 3 | 32 | 256 | 1,616 | 2,024 | 1,024 |
| | 05-55 | <4 | <4 | 64 | 405 | 1,618 | 1,024 | 512 |
| | Geometric average | <4 | 2 | 81 | 751 | 1,750 | 1,290 | 698 |

### Example 7: Examination of immunogenicity of combination live vaccines for oral administration on dogs carrying maternal antibodies

When puppies carrying a high level of maternal antibodies are vaccinated, vaccine viruses are disadvantageously neutralized, and immunity is not established. In recent years, some commercially available vaccines have elevated virus content to overcome the problem of the high level of maternal antibodies.

The present inventors have hypothesized that oral administration (i.e., along the natural route of infection) of the produced vaccine viruses instead of injection may overcome a low level of maternal antibodies and result in antibody response.

In this example, accordingly, vaccine viruses were administered orally to puppies carrying maternal antibodies, antibody titer was measured, and immunogenicity thereof was examined.

### Materials and method

### (1) Test animals

Ten healthy roughly 7- to 8-week-old beagle puppies and carrying maternal antibodies at the time of vaccine virus administration were adopted from Nosan Corporation (Table 5). Test dogs were individually raised in isolators.

**Table 5**

| List of test dogs | | |
|---|---|---|
| Dog number | Date of birth (age in week at the initiation of test) | Sex |
| IA35G | May 12, 2007 (8-week-old) | ♀ |
| JA55G | May 13, 2007 (8-week-old) | ♀ |
| QA45G | May 16, 2007 (7-week-old) | ♂ |
| QA65G | May 16, 2007 (7-week-old) | ♀ |
| QA75G | May 16, 2007 (7-week-old) | ♀ |
| RA45G | May 16, 2007 (7-week-old) | ♀ |
| SA45G | May 17, 2007 (7-week-old) | ♀ |
| SA55G | May 17, 2007 (7-week-old) | ♀ |
| LA45G | May 13, 2007 (8-week-old) | ♂ |
| MA55G | May 13, 2007 (8-week-old) | ♀ |

### (2) Test vaccines

Solutions of distemper viruses (CDV), canine adenoviruses (type 2) (CAV2), and canine parvoviruses (CPV), which had been attenuated at the Central Research Laboratories, Nippon Zenyaku Kogyo Co., Ltd., were mixed to prepare test vaccines. The virus contents are as shown below (Table 6).

The distemper virus 95-54/666 strains were obtained by subculturing the distemper virus 95-54 strains for 24 passages in Vero cells, the adenovirus type 2 F1-HP strains were obtained by subculturing the adenovirus type 2 F1 strains for 105 passages in MDCK cells, and the parvovirus F3/666 strains were obtained by subculturing the parvovirus F3 strain for 68 passages in Vero cells.

**Table 6**

| Virus content in test vaccine | | |
|---|---|---|
| Virus | Strain | Content per dog |
| CDV | 95-54/666 strain | 10^{4.50} TCID₅₀/mL |
| CAV2 | F1-HP strain | 10^{3.33} TCID₅₀/mL |
| CPV | F3/666 strain | 10^{4.67} TCID₅₀/mL |

### (3) Test method

The test schedule is shown in Table 7. After the strains were acclimated for 3 days after introduction, the first administration of vaccines virus was carried out at week 0. Oral administration was carried out by taking 1 ml of virus solution in a syringe and having the test dog drink the same. Three weeks thereafter, the same virus solution was orally administered. The blood serum were sampled every week from the second week before the first administration to the second week post second administration (a total of 6 times), and CDV and CAV2 neutralizing antibody titers and CPV HI values were measured. Positive antibody response was evaluated as prohibitive when the antibody titers were elevated to levels equivalent to or higher than those before vaccination.

**Table 7**

| Test schedule | | | | | | | |
|---|---|---|---|---|---|---|---|
| Test item | adoption | 0W | 1W | 2W | 3W | 4W | 5W |
| adoption | 7/3 | | | | | | |
| Virus administration | | 7/6(1^{st}) | | | 7/27(2^{nd}) | | |
| Blood sampling | | 7/6 | 7/13 | 7/20 | 7/27 | 8/3 | 8/10(completion) |
| Clinical observation | | | | every day | | | |

### Results and discussion

### 1) CDV neutralizing antibody titer

Table 8 shows transition in CDV neutralizing antibody titers in the test dogs after vaccine virus administration. Table 9 shows antibody responses in accordance with the level of maternal antibodies carried by each test dogs before vaccination. All test dogs carried maternal antibodies (1:4 to 1:22) before the first administration. At a week after the first vaccination, 9 of 10 dogs became antibody-negative, the other dogs were converted to become antibody-positive except for 1 dog in the second week, and the level of maternal antibodies became 1:512 to 1:2,048 two weeks after the second vaccination (i.e., upon completion of the test) (Table 8).

When the level of maternal antibodies was 1:8 or lower, the antibody response reached 100% as a result of the first vaccination (7 of 7 test dogs). While such level was 1:10 or higher, antibody response was not observed in 1 out of 3 test dogs after the first vaccination, but all the test dogs were converted to become antibody-positive after the second vaccination (Table 9).

**Table 8**

| Transition in CDV-neutralizing antibody titer | | | | | | |
|---|---|---|---|---|---|---|
| Dog number | Number of weeks after vaccination | | | | | |
| | 0W | 1W | 2W | 3W | 4W | 5W |
| | 1st Vac. | | | 2nd Vac. | | |
| IA35G | 8 | <4 | 3 | 162 | 648 | 2,048 |
| JA55G | 10 | <4 | <4 | <4 | <4 | 1,024 |
| QA45G | 8 | <4 | 41 | 162 | 1,024 | 1,024 |
| QA65G | 4 | <4 | 16 | 162 | 648 | 1,024 |
| QA75G | 8 | <4 | 8 | 41 | 256 | 512 |
| RA45G | 6 | <4 | 32 | 128 | 648 | 1,024 |
| SA45G | 10 | <4 | 32 | 128 | 1,618 | 2,048 |
| SA55G | 22 | 6 | 16 | 128 | 512 | 512 |
| LA45G | 4 | <4 | 64 | 256 | 2,048 | 2,048 |
| MA55G | 4 | <4 | 8 | 32 | 405 | 2,048 |

**Table 9**

| Antibody response in accordance with antibody titer before virus administration | | | |
|---|---|---|---|
| Virus | Antibody titer before administration | Number of times of vaccination | |
| | | Once | Twice |
| | 4 | 3/3 (100) * | 3/3 (100) |
| | ∼8 | 4/4 (100) | 4/4 (100) |
| CDV | ∼16 | 1/2 ( 50) | 2/2 (100) |
| | ∼32 | 1/1 (100) | 1/1 (100) |
| | Total | 9/10 ( 90) | 10/10 (100) |
| CAV2 | 4 | 1/1 (100) | 1/1 (100) |
| | ∼8 | 1/1 (100) | 1/1 (100) |
| | ∼16 | 0/1 ( 0) | 1/1 (100) |
| | ∼32 | 2/3 ( 67) | 3/3 (100) |
| | ∼64 | 1/2 ( 50) | 2/2 (100) |
| | ∼128 | 1/2 ( 50) | 2/2 (100) |
| | Total | 6/10 ( 60) | 10/10 ( 100) |
| CPV | <8 | 2/2 (100) | 2/2 (100) |
| | ∼16 | 2/2 (100) | 2/2 (100) |
| | ∼32 | 0/3 ( 0) | 2/3 ( 67) |
| | ∼64 | 0/1 ( 0) | 1/1 (100) |
| | ∼128 | 0/2 ( 0) | 1/2 ( 50) |
| | Total | 2/8 ( 25) | 6/8 ( 75) |

| | | | |
|---|---|---|---|
| * Number of dogs (%) | | | |

### 2) CAV2 neutralizing antibody titer

Table 10 shows transitions in CAV2 neutralizing antibody titers in test dogs after vaccine virus administration. Table 9 shows antibody response in accordance with the levels of maternal antibodies carried before vaccination. All test dogs carried maternal antibodies (1:4 to 1: 128) before the first administration. All the test dogs were converted to become antibody-positive upon completion of the test, and the range of antibody titers was between 1:64 and 1:6,472.

When the level of maternal antibodies was 1:8 or lower, the antibody response reached 100% as a result of the first vaccination (2 out of 2 test dogs). While such level was 1:10 or higher, lowering in antibody response was observed, but all the test dogs were converted to become antibody-positive after the second vaccination (Table 10).

**Table 10**

| Transition in CAV2-neutralizing antibody titer | | | | | | |
|---|---|---|---|---|---|---|
| Dog number | Number of weeks after vaccination | | | | | |
| | 0W | 1W | 2W | 3W | 4W | 5W |
| | 1st Vac. | 2nd Vac. | | | | |
| IA35G | 10 | 8 | <4 | <4 | <4 | 6,472 |
| JA55G | 32 | 32 | 64 | 256 | 162 | 512 |
| QA45G | 41 | 41 | 25 | 32 | 10 | 2,048 |
| QA65G | 41 | 32 | 128 | 101 | 162 | 256 |
| QA75G | 128 | 128 | 101 | 162 | 256 | 405 |
| RA45G | 8 | 8 | 64 | 101 | 101 | 128 |
| SA45G | 32 | 25 | 162 | 101 | 128 | 64 |
| SA55G | 128 | 64 | 32 | 10 | 10 | 405 |
| LA45G | 4 | 6 | 512 | 2,048 | 405 | 648 |
| MA55G | 25 | 16 | 8 | 6 | <4 | 2,048 |

### 3) CPV neutralizing antibody titer

Table 11 shows transitions in CPV-HI antibody titers in test dogs after vaccine virus administration. Table 9 shows antibody response in accordance with the level of maternal antibodies carried before vaccination. Since 2 out of the 10 test dogs were antibody-negative at the time of vaccination, they were not included in the data regarding the dogs carrying maternal antibodies. For reference, these two test dogs were converted to become antibody-positive 1 week after the first vaccination. The other 8 dogs carried 1:16 to 1:128 maternal antibodies. Upon completion of the test, the antibody response rate was 75% (6 out of 8 dogs), and the range of antibody titers were between 1:128 and 1:2,048.

When the level of maternal antibodies was 1:6 or lower, the antibody response reached 100% as a result of the first vaccination (2 out of 2 test dogs). When such level was 1:32 or higher, however, antibody response was not observed in any of the test dogs, and the antibody response reached 75% (4 out of 6 dogs) after the second vaccination.

In general, immunogenicity of vaccine strains and the amounts of viruses to be administered are known to be important in order to overcome a high level of maternal antibodies. The CPV content of the vaccines produced in the example is lower than that of commercially available vaccines. Thus, antibody response was difficult to attain in an individual carrying a high level of maternal antibodies. Thus, the vaccines may immunize a puppy carrying a high level of maternal antibodies by increasing the virus content thereof.

**Table 11**

| Transition in CPV HI antibody titers | | | | | | |
|---|---|---|---|---|---|---|
| Dog number | Number of weeks after vaccination | | | | | |
| | 0W | 1W | 2W | 3W | 4W | 5W |
| | 1st Vac. | 2nd Vac. | | | | |
| IA35G | 16 | 128 | 1,024 | 512 | 512 | 512 |
| JA55G | 32 | 16 | 16 | <8 | <8 | <8 |
| QA45G | 128 | 64 | 64 | 32 | 64 | 32 |
| QA65G | 64 | 32 | 32 | 32 | 32 | 1,024 |
| QA75G | 128 | 128 | 64 | 32 | 128 | 32 |
| RA45G | 32 | 32 | 32 | 8 | 64 | 512 |
| SA45G | 16 | 16 | 256 | 512 | 1,024 | 512 |
| SA55G | 32 | 32 | 16 | <8 | 128 | 2,048 |
| LA45G | <8 | 256 | 2,048 | 2,048 | 512 | 1,024 |
| MA55G | <8 | 128 | 1,024 | 1,024 | 1,024 | 2,048 |

The results of the example demonstrate that antibody response against viruses was attained via one or two vaccinations and that oral administration of such vaccines is effective on puppies carrying maternal antibodies.

### Industrial Applicability

The vaccines against canine distemper virus infections, canine adenovirus type 2 infections, and canine parvovirus infections according to the present invention, which can be orally administered, are useful for protection or treatment of dogs against such infections of such infections.

### Sequence Listing Free Text

SEQ ID NOs: 16 to 25: description of artificial sequences: primers

All publications, patents, and patent applications cited herein are incorporated herein by reference in their entirety.

### PH-3297-PCT

### PCT

| | | |
|---|---|---|
| 0-1 | Form PCT/RO/134(SAFE) Indications Relating to Deposited Microorganism(s) or Other Biological Material (PCT Rule 13bis) | |
| 0-1-1 | Prepared Using | JPO-PAS 0341 |
| 0-2 | International Application No. | |
| 0-3 | Applicant's or agent's file reference | PH-3297-PCT |
| | | |
| 1 | The indications made below relate to the deposited microorganism(s) or other biological material referred to in the description on: | |
| 1-1 | Paragraph | 0068 |
| 1-3 | Identification of deposit | |
| 1-3-1 | Name of depositary institution | ECACC European Collection of Cell Cultures |
| 1-3-2 | Address of depositary institution | Centre for Emergency Preparedness and Response, The Health Protection Agency, Porton Down, Salisbury, Wiltshire, SP4 0JG, UK . 16 July 2007 (16.07.2007) |
| 1-3-3 | Date of deposit | ECACC 07071601 |
| 1-3-4 | Accession Number | |
| 1-5 | Designated States for Which Indications are Made | all designations |
| 2 | The indications made below relate to the deposited microorganism(s) or other biological material referred to in the description on: | |
| 2-1 | Paragraph | 0068 |
| 2-3 | Identification of deposit | |
| 2-3-1 | Name of depositary institution | ECACC European Collection of cell cultures |
| 2-3-2 | Address of depositary institution | Centre for Emergency Preparedness and Response, Health Protection Agency, Porton Down, Salisbury, Wiltshire, SP4 0JG, UK 16 July 2007 (16.07.2007) |
| 2-3-3 | Date of deposit | ECACC 07071602 |
| 2-3-4 | Accession Number | |
| 2-5 | Designated States for Which Indications are Made | all designations |

### FOR RECEIVING OFFICE USE ONLY

| | | |
|---|---|---|
| 0-4 | This form was received with the international application: (yes or no) | |
| 0-4-1 | Authorized officer | |

### FOR INTERNATIONAL BUREAU USE ONLY

| | | |
|---|---|---|
| 0-5 | This form was received by the International Bureau on: | |
| 0-5-1 | Authorized officer | |

## Claims

1. A canine parvovirus F3 strain having a VP2 gene comprising the nucleotide sequence as shown in SEQ ID NO: 1 or a nucleotide sequence derived from the nucleotide sequence as shown in SEQ ID NO: 1 by deletion, substitution, or addition of one or several nucleotides.

2. The canine parvovirus F3 strain according to claim 1, which is the strain of any of the viruses (1) to (7) below:
(1) a virus having a genome comprising DNA containing the nucleotide sequence as shown in SEQ ID NO: 26;
(2) a virus having a genome comprising DNA containing a nucleotide sequence consisting of 500, 750, 1000, 1250, 1500, 1750, 2000, 2250, 2500, 2750, 3000, 3250, 3500, 3750, or 4000 continuous nucleotides of the nucleotide sequence as shown in SEQ ID NO: 26;
(3) a virus having a genome comprising DNA containing a nucleotide sequence consisting of nucleotides from 188, 200, or 630 to 4000 or 4017 of the nucleotide sequence as shown in SEQ ID NO: 26;
(4) a virus having a genome comprising DNA containing a nucleotide sequence consisting of nucleotides from 188, 200, or 630 to 480 of the nucleotide sequence as shown in SEQ ID NO: 26;
(5) a virus having a genome comprising DNA containing a nucleotide sequence consisting of nucleotides from 500 to 4000 or 4017 of the nucleotide sequence as shown in SEQ ID NO: 26;
(6) a virus having a genome comprising DNA that hybridizes under stringent hybridization conditions to DNA comprising a sequence complementary to DNA consisting of a nucleotide sequence of the genome according to any of (1) to (5) above; and
(7) a virus having a genome consisting of DNA having 95% or higher homology to DNA consisting of the nucleotide sequence of the genome according to any of (1) to (5) above.

3. A canine adenovirus type 2 F1 strain having an LITR E1A2 gene consisting of the nucleotide sequence as shown in SEQ ID NO: 5 or a nucleotide sequence derived from the nucleotide sequence as shown in SEQ ID NO: 5 by deletion, substitution, or addition of one or several nucleotides and having an RITR gene consisting of the nucleotide sequence as shown in SEQ ID NO: 7 or a nucleotide sequence derived from the nucleotide sequence as shown in SEQ ID NO: 7 by deletion, substitution, or addition of one or several nucleotides.

4. The canine adenovirus type 2 F1 strain deposited at the European Collection of Cell Cultures (ECACC) under Accession Number 07071601.

5. A canine distemper virus 95-54 strain having an H gene consisting of the nucleotide sequence as shown in SEQ ID NO: 9 or a nucleotide sequence derived from the nucleotide sequence as shown in SEQ ID NO: 9 by deletion, substitution, or addition of one or several nucleotides and having an F gene consisting of the nucleotide sequence as shown in SEQ ID NO: 12 or a nucleotide sequence derived from the nucleotide sequence as shown in SEQ ID NO: 12 by deletion, substitution, or addition of one or several nucleotides.

6. The canine distemper virus 95-54 strain deposited at the European Collection of Cell Cultures (ECACC) under Accession Number 07071602.

7. An attenuated canine parvovirus strain obtained by adapting the canine parvovirus F3 strain according to claim 1 or 2 in cultured cells to attenuate the same.

8. The attenuated canine parvovirus strain according to claim 7 comprising a nucleotide sequence derived from the nucleotide sequence of the VP2 gene as shown in SEQ ID NO: 1 by mutation of one or a plurality of, and preferably all of, nucleotides 885, 886, 892, 894, and 895.

9. The attenuated canine parvovirus strain according to claim 7 or 8 having a nucleotide sequence comprising 2 nucleotides inserted into a site between nucleotides 891 and 892 and/or 100 nucleotides inserted into a site between nucleotides 901 and 902.

10. An attenuated canine adenovirus type 2 strain obtained by adapting the canine adenovirus type 2 F1 strain according to claim 3 or 4 in cultured cells to attenuate the same.

11. The attenuated canine adenovirus type 2 strain according to claim 10, which has a nucleotide sequence derived from the nucleotide sequence of the LITR E1A2 gene as shown in SEQ ID NO: 5 by mutation of one or a plurality of nucleotides.

12. An attenuated canine distemper virus strain obtained by adapting the canine distemper virus 95-54 strain according to claim 5 or 6 in cultured cells to attenuate the same.

13. The attenuated canine distemper virus strain according to claim 12 having at least one of the mutations (1) to (3) below:
(1) mutation of nucleotides 394 and/or 523 of the F gene as shown in SEQ ID NO: 12;
(2) mutation of one or a plurality of, and preferably all of, nucleotides 56, 74, 78, 94, 100, 110, 116, 128, 135, 142, 156, 159, 174, 176, 182, 204, 218, 278, 306, 311, 320, 350, 374, 389, 398, 407, 416, 437, 446, 452, 456, 482, 485, 489, 495, 499, 531, 542, 548, 551, 553, 557, 558, 572, 598, 610, 614, 626, 629, 632, 647, 650, 659, 664, 680, 689, 698, 707, 716, 732, 737, 741, 753, 755, 776, 785, 788, 792, 812, 821, 850, 872, 884, 888, 914, 923, 929, 932, 935, 946, 947, 955, 957, 968, 977, 978, 983, 999, 1010, 1011, 1016, 1019, 1044, 1051, 1067, 1076, 1079, 1094, 1096, 1115, 1116, 1120, 1136, 1139, 1146, 1147, 1160, 1176, 1184, 1214, 1232, 1255, 1263, 1268, 1269, 1292, 1295, 1301, 1319, 1325, 1351, 1356, 1374, 1382, 1385, 1395, 1406, 1442, 1444, 1445, 1454, 1457, 1466, 1479, 1499, 1511, 1514, 1520, 1525, 1535, 1537, 1538, 1559, 1570, 1585, 1592, 1604, 1607, 1608, 1609, 1610, 1615, 1619, 1644, 1657, 1658, 1676, 1720, 1733, 1734, 1758, 1762, 1776, 1778, 1838, 1840, 1845, 1851, 1871, 1877, 1879, 1889, 1894, 1896, 1908, and 1911 of the H gene as shown in SEQ ID NO: 10; and
(3) mutation of one or a plurality of, and preferably all of, nucleotides 628, 634, 644, 648, 657, 681, 683, 718, 744, 748, 823, and 1406 of the H gene as shown in SEQ ID NO: 10.

14. A vaccine against canine virus infections comprising 1, 2, or 3 types of virus strains selected from the group consisting of the attenuated canine parvovirus strain according to any one of claims 7 to 9, the attenuated canine adenovirus type 2 strain according to claim 10 or 11, and the attenuated canine distemper virus strain according to claim 12 or 13.

15. A combination vaccine against canine virus infections comprising 3 types of virus strains selected from the group consisting of the attenuated canine parvovirus strain according to any one of claims 7 to 9, the attenuated canine adenovirus type 2 strain according to claim 10 or 11, and the attenuated canine distemper virus strain according to claim 12 or 13.

16. The combination vaccine against canine virus infections according to claim 14 or 15, wherein different virus strains do not interfere with each other.

17. The combination vaccine against canine virus infections according to claim 16, wherein the canine distemper virus and the canine parvovirus do not interfere with each other.

18. The vaccine against canine virus infections according to any one of claims 14 to 17, which is for oral administration.

19. The vaccine against canine virus infections according to any one of claims 14 to 18, which can immunize a puppy when it is vaccinated therewith up to 4 to 18 weeks after birth.

20. A method for protecting a dog from virus infections, which comprises orally administering 1, 2, or 3 types of virus strains selected from the group consisting of the attenuated canine parvovirus strain according to any one of claims 7 to 9, the attenuated canine adenovirus type 2 strain according to claim 10 or 11, and the attenuated canine distemper virus strain according to claim 12 or 13 to a dog.

21. The method for protecting a dog from virus infections according to claim 20, wherein different virus strains do not interfere with each other.

22. The method for protecting a dog from virus infections according to claim 20 or 21, wherein the canine distemper virus and the canine parvovirus do not interfere with each other.

23. The method for protecting a dog from virus infections according to any one of claims 20 to 22 comprising vaccination of a 4- to 18-week old puppy.


SEQUENCE LISTING

<110> NIPPON ZENYAKU KOGYO CO.,LTD

<120> A novel vaccine for canine infectious diseases

<130> PH-3297-PCT

<150> JP 2006-248522
<151> 2006-09-13

<160> 26

<170> PatentIn Ver. 2.1

<210> 1
<211> 1056
<212> DNA
<213> canine parvovirus

<400> 1
gaggcgtcta cacaagggcc atttaaaaca cctattgcag caggacgggg gggagcgcaa 60
acagatgaaa atcaagcagc agatggtgat ccaagatatg catttggtag acaacatggt 120
caaaaaacta ccacaacagg agaaacacct gagagattta catatatagc acatcaagat 180
acaggaagat atccagaagg agattggatt caaaatatta actttaacct tcctgtaaca 240
gatgataatg tattgctacc aacagatcca attggaggta aaacaggaat taactatact 300
aatatattta atacttatgg tcctttaact gcattaaata atgtaccacc agtttatcca 360
aatggtcaaa tttgggataa agaatttgat actgacttaa aaccaagact tcatgtaaat 420
gcaccatttg tttgtcaaaa taattgtcct ggtcaattat ttgtaaaagt tgcgcctaat 480
ttaacaaatg aatatgatcc tgatgcatct gctaatatgt caagaattgt aacttactca 540
gatttttggt ggaaaggtaa attagtattt aaagctaaac taagagcctc tcatacttgg 600
aatccaattc aacaaatgag tattaatgta gataaccaat ttaactatgt accaagtaat 660
attggaggta tgaaaattgt atatgaaaaa tctcaactag cacctagaaa attatattaa 720
catacttact atgtttttat gcttattaca tattatttta agattaatta aattacagca 780
tagaaatatt gtacttgtat ttgatatagg atttagaagg tttgttatat ggtatacaat 840
aactgtaaga aatagaagaa catttagatc atagttagta gtttgtttta taaaatgtat 900
tgtaaaccat taatgtatgt tgttatggtg tgggtggttg gttggtttgc ccttagaata 960
tgttaaggac caaaaaaatc aataaaagac atttaaaact aaatggcctc gtatactgtc 1020
tataaggtga actaacctta ccataagtat caatcg 1056

<210> 2

<211> 1158
<212> DNA
<213> canine parvovirus

<400> 2
gaggcgtcta cacaagggcc atttaaaaca cctattgcag caggacgggg gggagcgcaa 60
acagatgaaa atcaagcagc agatggtgat ccaagatatg catttggtag acaacatggt 120
caaaaaacta ccacaacagg agaaacacct gagagattta catatatagc acatcaagat 180
acaggaagat atccagaagg agattggatt caaaatatta actttaacct tcctgtaaca 240
gatgataatg tattgctacc aacagatcca attggaggta aaacaggaat taactatact 300
aatatattta atacttatgg tcctttaact gcattaaata atgtaccacc agtttatcca 360
aatggtcaaa tttgggataa agaatttgat actgacttaa aaccaagact tcatgtaaat 420
gcaccatttg tttgtcaaaa taattgtcct ggtcaattat ttgtaaaagt tgcgcctaat 480
ttaacaaatg aatatgatcc tgatgcatct gctaatatgt caagaattgt aacttactca 540
gatttttggt ggaaaggtaa attagtattt aaagctaaac taagagcctc tcatacttgg 600
aatccaattc aacaaatgag tattaatgta gataaccaat ttaactatgt accaagtaat 660
attggaggta tgaaaattgt atatgaaaaa tctcaactag cacctagaaa attatattaa 720
catacttact atgtttttat gcttattaca tattatttta agattaatta aattacagca 780
tagaaatatt gtacttgtat ttgatatagg atttagaagg tttgttatat ggtatacaat 840
aactgtaaga aatagaagaa catttagatc atagttagta gtttgtaata ttgtacttgt 900
atttgatata ggatttagaa ggtttgttat atggtataca ataactgtaa gaaatagaag 960
aacatttaga tcatagttag tagtttgttt tataaaatgt attgtaaacc attaatgtat 1020
gttgttatgg tgtgggtggt tggttggttt gcccttagaa tatgttaagg accaaaaaaa 1080
tcaataaaag acatttaaaa ctaaatggcc tcgtatactg tctataaggt gaactaacct 1140
taccataagt atcaatcg 1158

<210> 3
<211> 1241
<212> DNA
<213> canine parvovirus

<400> 3
gaggcgtcta cacaagggcc atttaaaaca cctattgcag caggacgggg gggagcgcaa 60
acatatgaaa atcaagcagc agatggtgat ccaagatatg catttggtag acaacatggt 120
caaaaaacta ccacaacagg agaaacacct gagagattta catatatagc acatcaagat 180
acaggaagat atccagaagg agattggatt caaaatatta actttaacct tcctgtaacg 240
aatgataatg tattgctacc aacagatcca attggaggta aaacaggaat taactatact 300
aatatattta atacttatgg tcctttaact gcattaaata atgtaccacc agtttatcca 360
aatggtcaaa tttgggataa agaatttgat actgacttaa aaccaagact tcatgtaaat 420
gcaccatttg tttgtcaaaa taattgtcct ggtcaattat ttgtaaaagt tgcgcctaat 480
ttaacaaatg aatatgatcc tgatgcatct gctaatatgt caagaattgt aacttactca 540

gatttttggt ggaaaggtaa attagtattt aaagctaaac taagagcctc tcatacttgg 600
aatccaattc aacaaatgag tattaatgta gataaccaat ttaactatgt accaagtaat 660
attggaggta tgaaaattgt atatgaaaaa tctcaactag cacctagaaa attatattaa 720
catacttact atggttttta tgtttattac atatcaacta gcacctagaa aaattatatt 780
aatatactta ctatggtttt tatgtttatt acatattatt ttaagattaa ttaaatacag 840
catagaaata ttgtacttgt atttgatata ggatttagaa gtttgttaga tggtatacaa 900
taactgtaag aaatagaaga acatttagat catagttagt aggtttgtta gatggtatac 960
aataactgta agaaatagaa gaacatttag atcatagtta gtaggtttgt tatatggtat 1020
acaataactg taagaaatag aagaacattt agatcatagt tagtagtttg ttttataaaa 1080
tgtattgtaa accattaatg tatgttgtta tggtgtgggt ggttggttgg tttgccctta 1140
gaatatgtta aggaccaaaa aaatcaataa aagacattta aaactaaatg gcctcgtata 1200
ctgtctataa ggtgaactaa ccttaccata agtatcaatc g 1241

<210> 4
<211> 1169
<212> DNA
<213> canine parvovirus

<400> 4
gaattcgccc ttgaggcgtc tacacaaggg ccatttaaaa cacctattgc agcaggacgg 60
gggggagcgc aaacagatga aaatcaagca gcagatggtg atccaagata tgcatttggt 120
agacaacatg gtcaaaaaac taccacaaca ggagaaacac ctgagagatt tacatatata 180
gcacatcaag atacaggaag atatccagaa ggagattgga ttcaaaatat taactttaac 240
cttcctgtaa cagatgataa tgtattgcta ccaacagatc caattggagg taaaacagga 300
attaactata ctaatatatt taatacttat ggtcctttaa ctgcattaaa taatgtacca 360
ccagtttatc caaatggtca aatttgggat aaagaatttg atactgactt aaaaccaaga 420
cttcatgtaa atgcaccatt tgtttgtcaa aataattgtc ctggtcaatt atttgtaaaa 480
gttgcgccta atttaacaaa tgaatatgat cctgatgcat ctgctaatat gtcaagaatt 540
gtaacttact cagatttttg gtggaaaggt aaattagtat ttaaagctaa actaagagcc 600
tctcatactt ggaatccaat tcaacaaatg agtattaatg tagataacca atttaactat 660
gtaccaagta atattggagg tatgaaaatt gtatatgaaa aatctcaact agcacctaga 720
aaattatatt aacatactta ctatgttttt atgcttatta catattattt taagattaat 780
taaattacag catagaaata ttgtacttgt atttgatata ggatttagaa ggtttgttat 840
atggtataca ataactgtaa gaaatagaag aacatttaga tcatagttag tagtttgtaa 900
tattgtactt gtatttgata taggatttag aaggtttgtt atatggtata caataactgt 960
aagaaataga agaacattta gatcatagtt agtagtttgt tttataaaat gtattgtaaa 1020
ccattaatgt atgttgttat ggtgtgggtg gttggttggt ttgcccttag aatatgttaa 1080
ggaccaaaaa aatcaataaa agacatttaa aactaaatgg cctcgtatac tgtctataag 1140
gtgaactaac cttaccataa gtatcaatc 1169

<210> 5
<211> 1907
<212> DNA
<213> Canine adenovirus type 2

<400> 5
catcatcaat aatatacagg acaaagaggt gtggcttaaa tttgggcgtt gcaaggggcg 60
gggtcatggg acggtcaggt tcaggttacg ccctggtcag ggtgttccca cgggaatgtc 120
cagtggcgtc aaaggcgtgg ttttacgaca gggcgagttc cgcggacttt tggccggcgc 180
cccgggtttt tgggcgttta ttgattttgc ggtttattgg gtggtgcttt taccactgtt 240
tgcggaagat ttagttgttt atggagctgg ttttggtgcc agttcctcca cgcctaatgt 300
caaagtttat gtcaatataa cagaaacact ctgttctctg tttacagcgc cccacccggt 360
ggtttttcgc cacgcctttg ggttaatttt atttccctat acgcggcctt aaattctcag 420
tgcagacgaa agaggactac tcttgagtgc gcagcgagaa gagttttctc ttcgctgtgt 480
ctcatatatt ctctgaaaaa tgaaatatac tattgtgccg gcgccgcgca atctccatga 540
ttatgtttta gagctactgg aagagtggca gccggactgc cttgactgtg agtatcctca 600
tggcagcccc tcgccgccta ctctgcacga tctttttgat gttgagctgg agacttctna 660
cagccctttt gtgggcctgt gtgattcctg tgcggaggct gacactgatt cgagtgcgag 720
cactgaggct gattttgggt ttagtccttt gtccactccg ccggtttcac ctattccacc 780
gcatcccacc tntcctgcta gcatttctga cgacatgttg ctgtgcttag aggaaatgcc 840
cacctttgat gacgaggacg aggttcgaag cgcggcgacc acctttgagc ggtgggaaaa 900
cacttttgac ccccatgtgg gtcctatttt tggctgtttg cgctgtgctt tttatcaaga 960
gcaggatgat aatgcacttt gtgggctttg ctatctaaag gcccttgccg aaggtaagtt 1020
ttaatttaaa tgtttgggca ggttaaatgt ttgggcaggt taaatgtttt aggtgtgtat 1080
tgatttttaa ttttgctttt tagtgccttt tgctttgcct gtacgttcag aacccgcttc 1140
ggctggagct gaggaggaag atgatgaagt tatttttgtg tctgccaaac ctgggggcag 1200
aaagaggtca gcagctactc cctgtgagcc agatggggcc agcaaacgcc cttgcgtgcc 1260
agagcctgag caaacagaac ctttggattt gtctttgaag ccacgcccga actaatctcc 1320
ttgagcacaa agcaataaag taatcttgtt taacaagttt gcctacattt gtggttttac 1380
ggggcggggc gaggagtata taatgccaaa agccagtgtc tgcttcatta agcttttaga 1440
ctgagctaag agcaggtagt atggaccctc ttaagatttg tgaaaactac cttactttta 1500
gagctataat taggggaagt actttgtcgc ctggattttt taggcggtgg tgttttcctg 1560
ccttggctga tgtggtgggc aatatagtgg tacaggagga aggcaggttt tggcaaattt 1620
tacctgaaaa ccacgcnttt tggggtcttt tgcgcagggg ctttactgtt gcttctttta 1680
ctgaaattat tacagcagct cagctggaaa atagaggtag acagttggcc tttttagctt 1740
ttatatcatt tttgctacgc aactggcctt ctgactctgt agtgcctgaa gctgacagac 1800
ttgacctggt ctgtgcgccg gcatggagca gaatgcagat atggagccag accgccaggt 1860
taatcaacga cctccaagat tccgtgctcg aggagcaggg gtccgcg 1907

<210> 6
<211> 1908

<212> DNA
<213> Canine adenovirus type 2

<400> 6
catcatcaat aatatacagg acaaacaggt gtggcttaaa tttgggcgtt gcaaggggcg 60
gggtcatggg acggtcaggt tcaggttacg ccctggtcag ggtgttccca cgggaatgtc 120
cagtggcgtc aaaggcgtgg ttttacgaca gggcgagttc cgcggacttt tggccggcgc 180
cccgggtttt tgggcgttta ttgattttgc ggtttattgg gtggtgcttt taccactgtt 240
tgcggaagat ttagttgttt atggagctgg ttttggtgcc agttcctcca cgcctaatgt 300
caaagtttat gtcaatataa cagaaacact ctgttctctg tttacagcgc cccacccggt 360
ggtttttcgc cacgcctttg ggttaatttt atttccctat acgcggcctt aaattctcag 420
tgcagacgaa agaggactac tcttgagtgc gcagcgagaa gagttttctc ttcgctgtgt 480
ctcatatatt ctctgaaaaa tgaaatatac tattgtgccg gcgccgcgca atctccatga 540
ttatgtttta gagctactgg aagagnggca gccggactgc cttgactgtg agtatcctna 600
tggcagcccc tcgccgccta ctntgcacga tctttttgat gttgagctgg agacttctna 660
cagccntttn gnggncctgt gtgattcctg tgcggaggct gacactgatt cgagtgcnag 720
cactgaggct gattttgggt ttagtccttt gtccactccg ccggtttnac ctattccacc 780
gnatcccacc tntcctgnta gcatttctga cnacatgttg ctgtgcttag aggaaatgcc 840
cacctttgat gacgaggacg aggttcgaag cgcggcgacc acctttgagc ggtgggaaaa 900
cacttttgac ccccatgtgg gtcctatttt tggctgtttg cgctgtgctt tttatcaaga 960
gcaggatgat aatgcacttt gtgggctttg ctatctaaag gcccttgccg aaggtaagtt 1020
ttaatttaaa tgtttgggca ggttaaatgt ttgggcaggt taaatgtttt aggtgtgtat 1080
tgatttttaa ttttgctttt tagtgccttt tgctttgcct gtacgttcag aacccgcttc 1140
ggctggagct gaggaggaag atgatgaagt tatttttgtg tctgccaaac ctgggggcag 1200
aaagaggtca gcagctactc cctgtgagcc agatggggcc agcaaacgcc cttgcgtgcc 1260
agagcctgag caaacagaac ctttggattt gtctttgaag ccacgcccga actaatctcc 1320
ttgagcacaa agcaataaag taatcttgtt taacaagttt gcctacattt gtggttttac 1380
ggggcggggc gaggagtata taatgccaaa agccagtgtc tgcttcatta agcttttaga 1440
ctgagctaag agcaggtagt atggaccctc ttaagatttg tgaaaactac cttactttta 1500
gagctataat taggggaagt actttgtcgc ctggattttt taggcggtgg tgttttcctg 1560
ccttggctga tgtggtgggc aatatagtgg nacaggagga aggcaggttt tggcaaattt 1620
tacctgaaaa ccacgcnttt tggggtcttt tgcgcagggg ctttactgnt gcttctttta 1680
ctgaaattat tacagcagct cagctggaaa atanaggtag acagttggcc nttttagctt 1740
ttatatcatt tttgctacgc aactggcctt ctgactctgt agtgcctgaa gctgacagac 1800
ntgacctggt ctgtgcgccg gcatggagca gaatgcagat atggagccag accgccaggt 1860
taatcaacga cctccnagat tccgtgctcg aggagcaggg ggccgtna 1908

<210> 7
<211> 488
<212> DNA
<213> Canine adenovirus type 2

<400> 7
catagacact cctaaagctg ccatagtact cacggacggc tggctgtcaa aggagagcta 60
tgaggatgaa atgccaagca cggcgtttat atagtcctca aagtagggcg tgtggaaaac 120
gaaaaggaat ataacggggc gtttgaggaa gtggtgccaa gtacagtcat aaattgtggg 180
cgcgtggtaa atgttaagtg cagtttccct ttggcggttg gcccggaaag ttcacaaaaa 240
gtacagcacg tccttgtcac cgtgtcaacc acaaaaccac aaataggcac aacgcccaaa 300
aacccggggc gccggccaaa agtccgcgga actcgccctg tcgtaaaacc acgcctttga 360
cgccactgga cattcccgtg ggaacaccct gaccagggcg taacctgaac ctgaccgtcc 420
catgaccccg ccccttgcaa cgcccaaatt taagccacac ctctttgtcc tgtatattat 480
tgatgatg 488

<210> 8
<211> 488
<212> DNA
<213> Canine adenovirus type 2

<400> 8
catagacact cctaaagctg ccatagtact cacggacggc tggctgtcaa aggagagcta 60
tgaggatgaa atgccaagca cggcgtttat atagtcctca aagtagggcg tgtggaaaac 120
gaaaaggaat ataacggggc gtttgaggaa gtggtgccaa gtacagtcat aaattgtggg 180
cgcgtggtaa atgttaagtg cagtttccct ttggcggttg gcccggaaag ttcacaaaaa 240
gtacagcacg tccttgtcac cgtgtcaacc acaaaaccac aaataggcac aacgcccaaa 300
aacccggggc gccggccaaa agtccgcgga actcgccctg tcgtaaaacc acgcctttga 360
cgccactgga cattcccgtg ggaacaccct gaccagggcg taacctgaac ctgaccgtcc 420
catgaccccg ccccttgcaa cgcccaaatt taagccacac ctctttgtcc tgtatattat 480
tgatgatg 488

<210> 9
<211> 1961
<212> DNA
<213> canine distemper virus

<400> 9
gaattcgccc ttagggctca ggtagtccag caatgctctc ctaccaagac aaggtgggtg 60
ccttctataa ggacaatgca agagctaatt catccaagct gtccccagtg acagaagagc 120
aagggggaag gagaccaccc tatttgctgt ttgcccttct catcctactg attggaatcc 180
tggccttact tgccatcact ggagttcgat ttcaccaagt atcaactagt aatatggaat 240
ttagcagatt gctgaaagag gatatggaga aatcagaggc cgtacatcac caagtcatag 300
atgtcttgac accgctcctc aaaattattg gagatgagat tgggttacgg ttgccacaaa 360

aactaaacga gatcaaacaa tttattcttc aaaagacaaa cttcttcaat ccgaacaggg 420
aattcgactt ccgcgatctc cactggtgta ttaacccacc tagcaagatc aaggtgaatt 480
ttactaatta ctgcgataca gttggggtca aaaaatctat tgcatcggca gcaaatccca 540
tcattttatc agcactctcc ggagccagag gtgacatatt cccgccatac agatgcagtg 600
gagctactac ttcagtaggc agagtattcc ccctatccgt atcattatcc atgtctttaa 660
tatcaagaac atcagagata atcaatatgc taaccgctat atcagacgga gtgtatggta 720
aaacttattt gctagtgcct gattatattg aaggggagtt cgactcgcaa gagattcgag 780
tctttgagat agggtttatc aaacggtggc tgaatgacat gcctttactc cagacaacca 840
actatatggt cctcccggag acttccaaag ccaaggtatg tactatagca gtgggcgagc 900
tgacactggc ttccttgtgt gtagatgaga gcaccgtatt gttgtatcat gacagcaatg 960
gttcacaaaa tggtattctg gtagtgacgt tgggaatatt tggggcaaca cctatggatc 1020
aagttgaaga ggtgatacct gtcgctcacc catcagtgga gagaatacat ataacaaatc 1080
accgtgggtt cataaaagat tcaatagtaa cctggatggt gcctgcattg gtctctgaga 1140
aacaagagga gcaaaaaaat tgtctggagt cggcttgtca aagaaaatcc taccctatgt 1200
gcaaccaaac gtcatgggaa ccctttggag gaggacagtt gccttcttat gggcggttga 1260
cattacttct agatccaagc attgaccttc aacttaacat atcatttaca tatggtccgg 1320
ttatactgaa cggagacggt atggattatt atgaaagccc acctttggac tccggatggc 1380
ttaccgttcc tcccaagaac ggaacagtcc ttggattgat aaacaaagca agtagaggag 1440
accagttcac tgtgaccccc catgtgttga catttgcgcc cagggaatca agtggaaatt 1500
gttatttacc cattcaaaca tcccagatta tggataaaga tgtccttact gagtccaatt 1560
tagtggtgtt acctacacag aattttagat atgtcatagc aacatatgat atatcccggg 1620
gcgatcgtgc aattgtttat tatgtttatg acccaatccg gacgatttcc tatacgtacc 1680
catttaggct aactaccaag ggtagacctg atttcctaag gattgaatgt tctgtgtggg 1740
atgacgattt gtggtgtcac caattttacc gattcgaggc taacatcact aactctacaa 1800
ccagtgttga gaatttagtc cgtataagat tctcatgtaa ccgttcaaaa ccttgatagt 1860
atgatgatac acatctcaat tgaacttaag gatgatgact gtggtaagaa atcccttacc 1920
gacgattgaa ttaaaccatc tctagcatta agggcgaatt c 1961

<210> 10
<211> 1961
<212> DNA
<213> canine distemper virus

<400> 10
   gaattcgccc ttagggctca ggtagtccag caatgctctc ctaccaagac aaggtgggtg 60
   ccttctacaa ggacaatgca agagccaatc catccaagct gtccctagtg aaagaagagc 120
   atgggggcag gagaccacct tatttgttgt ttgtccttct catcctattg gttggaatcc 180
   tggccctgct tgctatcact ggagttcgat ttcacaaagt atcaactagc aatatggaat 240
   ttagcagatt gctgaaagag gatatggaga aatcagaggc cgtacatcat caagtcatag 300
   atgtcttgac accgctcttc aagattattg gggatgagat tgggttacgg ttgccacaaa 360
   agctaaacga gatcaaacaa tttatccttc aaaagacaaa tttcttcaac ccgaacagag 420
   aattcgattt ccgcgatctc cactggtgca ttaacccgcc tagtaaggtc aaggtgaatt 480
   ttactaatta ctgtgagaca attgggatca gaaaatctat tgcatcggca gcaaatccca 540
   tccttttatc agccctctct gggggcagga gtgacatatt cccaccatac agatgcagtg 600
   gagctactat ttcagtaggc aaagttttcc ccctatcagt ctcgttatcc atgtctttga 660
   tctcaagaac ctcagtgata atcaatatgc tgaccgctat ctcagacggc gtgtatggca 720
   aaacttactt gctagtgcct gatgatatag aacgggagtt cgacactcaa gagattcgag 780
   tctttgaaat agggttcatt aaaaggtggc tgaatgacat gccattactc caaacaacca 840
   actatatggt cctcccggag aattccaaag ccaaggtatg taccatagca gtgggtgagt 900
   tgacactggc ttccttgtgt gtagaagaga gcactgtatt attataccat gacagcaggg 960
   gttcacgaga tggtattcta gtagtgacac tggggatatt tggggcaaca tctatggatc 1020
   acattgagga agtgatacct gtcgctcacc catcaatgga gaaaatacat ataacaaacc 1080
   accgtggttt tataaaagat tcaattgcaa cctggatggt gcctgccctg gcctctgaga 1140
   aacaagaaga acaaaaaggt tgtctggagt cagcttgtca aagaaaaacc taccccatgt 1200
   gcaaccaaac gtcatgggaa cccttcggag gaggacagtt gccatcttat gggcggttga 1260
   cattacctct agatgcaagt gttgaccttc aacttaacat atcgttcaca tacggtccgg 1320
   ttatactgaa tggagatggt atggattatt atgaaagccc acttttgaac tccggatggc 1380
   ttaccattcc tcctaaaaac ggaacaatcc ttggattaat aaacaaagca agtagaggag 1440
   accagttcac tgtaataccc catgtattaa catttgcacc cagggaatca ggtggaaatt 1500
   gttatttacc tattcaaaca tctcaaatta tagatagaga tgtcctcatc gagtccaatt 1560
   tagtggtgtt gcctacacag agttttagat atgtcagagc aacgtatgat atatcacgaa 1620
   atgatcatgc gattgtttat tatgtttatg acccattccg gacgattttt tatacgtacc 1680
   catttagact aactaccaag ggtagacctg atttcctaag gattgaatgt tttgtgtggg 1740
   atgataattt gtggtgtcac caattttaca gatacgaggc taacatcgcc aactctacaa 1800
   ccagtgttga gaatttagtc cgtataagat tctcatgtaa ccgttcaaat ccctgacagt 1860
   ataatgatac acatctcaat tggacttagg catgatgact atggtgaaaa atcccttaca 1920
   gatgattgaa ttaaaccatc tctagcatta agggcgaatt c 1961

<210> 11
<211> 1937
<212> DNA
<213> canine distemper virus

<400> 11
agggctcagg tagtccagca atgctctcct accaagacaa ggtgggtgcc ttctacaagg 60
acaatgcaag agccaatcca tccaagctgt ccctagtgaa agaagagcat gggggcagga 120
gaccacctta tttgttgttt gtccttctca tcctattggt tggaatcctg gccctgcttg 180
ctatcactgg agttcgattt cacaaagtat caactagcaa tatggaattt agcagattgc 240
tgaaagagga tatggagaaa tcagaggccg tacatcatca agtcatagat gtcttgacac 300
cgctcttcaa gattattggg gatgagattg ggttacggtt gccacaaaag ctaaacgaga 360
tcaaacaatt tatccttcaa aagacaaatt tcttcaaccc gaacagagaa ttcgatttcc 420
gcgatctcca ctggtgcatt aacccgccta gtaaggtcaa ggtgaatttt actaattact 480

gtgagacaat tgggatcaga aaatctattg catcggcagc aaatcccatc cttttatcag 540
ccctctctgg gggcaggagt gacatattcc caccatacag atgcagtgga gctactattt 600
cagtaggcaa agttttcccc ctatcagcct cgtaatccat gtccttggtc tcaagaccct 660
cagtgataat caatatgctg ccggctatct cagacggcgt gtatggcaaa acttactggc 720
tagtgcctga tgatatagaa cggaagtccg acactcaaga gattcgagtc tttgaaatag 780
ggttcattaa aaggtggctg aatgacatgc cattactcca aataaccaac tatatggtcc 840
tcccggagaa ttccaaagcc aaggtatgta ccatagcagt gggtgagttg acactggctt 900
ccttgtgtgt agaagagagc actgtattat tataccatga cagcaggggt tcacgagatg 960
gtattctagt agtgacactg gggatatttg gggcaacatc tatggatcac attgaggaag 1020
tgatacctgt cgctcaccca tcaatggaga aaatacatat aacaaaccac cgtggtttta 1080
taaaagattc aattgcaacc tggatggtgc ctgccctggc ctctgagaaa caagaagaac 1140
aaaaaggttg tctggagtca gcttgtcaaa gaaaaaccta ccccatgtgc aaccaaacgt 1200
catgggaacc cttcggagga ggacagttgc catcttatgg gcggttgaca ttacctctag 1260
atgcaagtgt tgaccttcaa cttaacatat cgttcacata cggtccggtt atactgaatg 1320
gagatggtat ggattattat gaaagcccac ttttgaactc cggatggctt accattcctc 1380
ctaaaaacgg aacaatcctt ggattgataa acaaagcaag tagaggagac cagttcactg 1440
taatacccca tgtattaaca tttgcaccca gggaatcagg tggaaattgt tatttaccta 1500
ttcaaacatc tcaaattata gatagagatg tcctcatcga gtccaattta gtggtgttgc 1560
ctacacagag ttttagatat gtcagagcaa cgtatgatat atcacgaaat gatcatgcga 1620
ttgtttatta tgtttatgac ccattccgga cgatttttta tacgtaccca tttagactaa 1680
ctaccaaggg tagacctgat ttcctaagga ttgaatgttt tgtgtgggat gataatttgt 1740
ggtgtcacca attttacaga tacgaggcta acatcgccaa ctctacaacc agtgttgaga 1800
atttagtccg tataagattc tcatgtaacc gttcaaatcc ctgacagtat aatgatacac 1860
atctcaattg gacttaggca tgatgactat ggtgaaaaat cccttacaga tgattgaatt 1920
aaaccatctc tagcatt 1937

<210> 12
<211> 2114
<212> DNA
<213> canine distemper virus

<400> 12
gaattcgccc ttgccaacag gtcaaccagg tccaccagcc cgaggccggg caggaacccc 60
cacaaacagc caagccccat gcacaaggca atccccaaaa gctccaaaac ccacacacat 120
acccaacaaa accgcccctc acaacccagc accggacccg acgagaccag gacctcccga 180
gcacgacaca gcataacgtc agctcagcga tccacgcact atgatcctcg aacattggac 240
agacccgtct cctacaccat gaacaggacc aggtctcgca agcaaaccag ctactgattg 300
gagaccatct cagttcacgg aaaccacgag gctattatcc agcacatgcc agagagtgtc 360
tccaaaggag cgagatccca gatcgaaagg cggcgaccca atgcaatcaa ctcaggctct 420
cagtgctcct ggttaatcct gtggtgcctc ggaatagcca gtctctttct ttgttccaag 480
gctcagatac gttggaataa tttgtcaact attgggatta ttaggactga tagtgtccat 540

tataagatca tggctaggcc cagtcaccag tacttggtca taaaactgat gcctaatgtt 600
tcacttatag ataattgtac caaagcagaa ttaggtgagt atgagaaatt attgaattca 660
gtcctcgaac caatcaacca agctttgact ctaatgacca agaatgtgaa gcccttgcag 720
tcattagggt cagctaggag acaaaggcgt tttgcaggag tagtacttgc aggtgcagct 780
ttaggagtgg ctacagctgc acaaatcact gcaggaatag ctttacatca atccaacctc 840
aatgctcaag caatccaatc tcttagaacc agccttgaac agtctaacaa agctattgaa 900
gaaattaggg aggctaccca agaaaccgtc attgccgttc agggagtcca ggattacgtc 960
aacaacgaac tcgtccctgc tatgcaacat atgtcatgtg aattagttgg gcagagatta 1020
gggttgaaac tgcttaggta ttatactgag ttattgtcaa tatttggccc gagtttacgt 1080
gaccctattt cagccgagat atcaattcag gcactgagtt atgctcttgg aggagaaatt 1140
cataagatac tcgagaagtt gggatactct ggaggtgata tgattgcaat cttggagagt 1200
cgggggataa aaacaaaaat aactcatgtt gatattcccg ggaaattcat catcctaagt 1260
atctcttacc caactttatc agaagtcaag ggggttatag tccacagact ggaagcagtt 1320
tcctacaaca taggatcaca agagtggtac accactgtcc cgaggtatat tgcaactaat 1380
ggttacttaa tatctaattt tgatgagtca tcctgtgtat tcgtctcaga gtcagccatt 1440
tgtagccaga actccctgta ccccatgagc ccactcttac aacaatgtat taggggcgac 1500
acttcatctt gtgctcgaac cttggtatct gggactatgg gcaacaaatt tattctgtca 1560
aaaggtaata tcgtcgcaaa ttgtgcgtct atactatgta agtgttatag cacaagcaca 1620
attattaatc agagtcctga taagttgctg acattcattg cctccgatac ctgcccactg 1680
gttgaaatag atggtgtaac tatccaagtt ggaggcaggc aataccctga tatggtatat 1740
gaaagcaaag ttgccttagg ccctgctata tcacttgaga ggttagatgt aggtacaaat 1800
ttagggaacg cccttaagaa actggatgat gctaaggtac tgatagactc ctctaaccag 1860
atccttgaga cggttaggcg ctcttccttt aattttggca gtctcctcag cgttcctata 1920
ttaagtggta cagccctggc tttgctgttg ctgatttact gttgtaaaag acgctaccaa 1980
cagacactca agcggaatac taaggtcgat ccggcattta aacctgatct aactggaact 2040
tcgaaatcct atgtgagatc actctgaagt attctggtca tatatctcgc ttattgccag 2100
gaaagggcga attc 2114

<210> 13
<211> 2114
<212> DNA
<213> canine distemper virus

<400> 13
gaattcgccc ttgccaacag gtcaaccagg tccaccagcc cgaggccggg caggaacccc 60
cacaaacagc caagccccat gcacaaggca atccccaaaa gctccaaaac ccacacacat 120
acccaacaaa accgcccctc acaacccagc accggacccg acgagaccag gacctcccga 180
gcacgacaca gcataacgtc agctcagcga tccacgcact atgatcctcg aacattggac 240
agacccgtct cctacaccat gaacaggacc aggtctcgca agcaaaccag ctactgattg 300
gagaacatct cagttcacgg aaaccacgag gctattatcc agcacatgcc agagagtgtc 360
tccaaaggag cgagatccca gatcgaaagg cggngaccca atgcaatcaa ctcaggctct 420

cagtgctcct ggttaatcct gtggtgcctc ggaatagcca gtctctttct ttgttccaag 480
gctcagatac gttggaataa tttgtcaact attgggatta ttgggactga tagtgtccat 540
tataagatca tggctaggcc cagtcaccag tacttggtca taaaactgat gcctaatgtt 600
tcacttatag ataattgtac caaagcagaa ttaggtgagt atgagaaatt attgaattca 660
gtcctcgaac caatcaacca agctttgact ctaatgacca agaatgtgaa gcccttgcag 720
tcattagggt cagctaggag acaaaggcgt tttgcaggag tagtacttgc aggtgcagct 780
ttaggagtgg ctacagctgc acaaatcact gcaggaatag ctttacatca atccaacctc 840
aatgctcaag caatccaatc tcttagaacc agccttgaac agtctaacaa agctattgaa 900
gaaattaggg aggctaccca agaaaccgtc attgccgttc agggagtcca ggattacgtc 960
aacaacgaac tcgtccctgc tatgcaacat atgtcatgtg aattagttgg gcagagatta 1020
gggttgaaac tgcttaggta ttatactgag ttattgtcaa tatttggccc gagtttacgt 1080
gaccctattt cagccgagat atcaattcag gcactgagtt atgctcttgg aggagaaatt 1140
cataagatac tcgagaagtt gggatactct ggaggtgata tgattgcaat cttggagagt 1200
cgggggataa aaacaaaaat aactcatgtt gatattcccg ggaaattcat catcctaagt 1260
atctcttacc caactttatc agaagtcaag ggggttatag tccacagact ggaagcagtt 1320
tcctacaaca taggatcaca agagtggtac accactgtcc cgaggtatat tgcaactaat 1380
ggttacttaa tatctaattt tgatgagtca tcctgtgtat tcgtctcaga gtcagccatt 1440
tgtagccaga actccctgta ccccatgagc ccactcttac aacaatgtat taggggcgac 1500
acttcatctt gtgctcgaac cttggtatct gggactatgg gcaacaaatt tattctgtca 1560
aaaggtaata tcgtcgcaaa ttgtgcgtct atactatgta agtgttatag cacaagcaca 1620
attattaatc agagtcctga taagttgctg acattcattg cctccgatac ctgcccactg 1680
gttgaaatag atggtgtaac tatccaagtt ggaggcaggc aataccctga tatggtatat 1740
gaaagcaaag ttgccttagg ccctgctata tcacttgaga ggttagatgt aggtacaaat 1800
ttagggaacg cccttaagaa actggatgat gctaaggtac tgatagactc ctctaaccag 1860
atccttgaga cggttaggcg ctcttccttt aattttggca gtctcctcag cgttcctata 1920
ttaagtggta cagccctggc tttgctgttg ctgatttact gttgtaaaag acgctaccaa 1980
cagacactca agcggaatac taaggtcgat ccggcattta aacctgatct aactggaact 2040
tcgaaatcct atgtgagatc actctgaagt attctggtca tatatctcgc ttattgccag 2100
gaaagggcga attc 2114

<210> 14
<211> 1378
<212> DNA
<213> canine distemper virus

<400> 14
gaattcgccc ttgatctgcc ggcaaagtaa gctctgcact tgccgccgag cttggcatca 60
ccaaggagga agctcagctg gtgtcagaaa tagcatccaa gacaacagag gaccggacga 120
ttcgagctac tggtcccaag cagtcccaaa ttaccttcct gcactcggaa agatccgaag 180
tcgctaatca acaacccccg accatcaaca agaggtccga aaaccaggga ggagacaaat 240
accccattca cttcagtgat gaaaggcttc cagggtatac cccagatgtc aacagttctg 300

aatggagtga gtcacgctat gacacccgaa ttatccaaga tgatggaaat gacgatgacc 360
ggaaatcgat ggaagcaatc gccaagatga ggatgcttac taagatgctc agtcaacctg 420
ggaccagtga agatagttct cctgtttata atgatagaga gctactcaat taaatattca 480
agaccagtct tgcatcagtc aacaattatc attctaaact cattataaaa aacttaggac 540
ccaggtccaa caaacccgat caaccattca tccgaccacc cgttctatcc ctaaatggca 600
gaggaacagg cctaccatgt cagcaaaggg ctggaatgcc tcaaagccct cagagagaat 660
cctcctgaca ttgaggagat tcaagaggtc agcagcatca gagaccaaac ctgcaaccca 720
ggccaagaga atggaaccac aggcatgcag gaagaggagg actctcagaa tctcgatgaa 780
tcacacgagc caacaaaagg atcaaactat gtcggccatg tactccaaaa taatccggga 840
tgtggagaaa gcaactctgt gtttgtggaa gcagagcagc cccctaaaga ggacatccaa 900
ccaggacctg gaatacgatg ttatcatgtt tatgatcaca gcggtgaaga ggttaaggga 960
atcgaagatg ctgacagtct cgtggtacct gcaggcactg tcggtaatcg aggattcgag 1020
agcggagaag gaagccctga tgatagcact gaggattctg gcgaagatta ttccgaagga 1080
aatgcttcat ctaactgggg atattctttc ggccttaaac cagacagggc agctgatgtg 1140
agcatgctga tggaagagga attaagtgct ctgctcaaga caagcagaaa tgtagggatt 1200
cagaaaaggg atgggaagac tctgcagttc ccacataatc ccgaaggtaa gacaagggtt 1260
tcggagtgtg gatccattaa aaaggacaca gaagagaggt cagtctcaca tggaatgggg 1320
atagttgctg gatcgacaag tggtgcaacc caatactagt aattaaaagg gcgaattc 1378

<210> 15
<211> 1379
<212> DNA
<213> canine distemper virus

<400> 15
gaattcgccc ttgatctgcc ggcaaagtaa gctctgcact tgccgccgag cttggcatca 60
ccaaggagga agctcagctg gtgtcagaaa tagcatccaa gacaacagag gaccggacga 120
ttcgagctac tggtcccaag cagtcccaaa ttaccttcct gcactcggaa agatccgaag 180
tcgctaatca acaacccccg accatcaaca agaggtccga aaaccaggga ggagacaaat 240
accccattca cttcagtgat gaaaggcttc cagggtatac cccagatgtc aacagttctg 300
aatggagtga gtcacgctat gacacccgaa ttatccaaga tgatggaaat gacgatgacc 360
ggaaatcgat ggaagcaatc gccaagatga ggatgcttac taagatgctc agtcaacctg 420
ggaccagtga agatagttct cctgtttata atgatagaga gctactcaat taaatattca 480
agaccagtct tgcatcagtc aacaattatc attctaaact cattataaaa aacttaggac 540
ccaggtccaa caaacccgat caaccattca tccgaccacc cgttctatcc ctaaatggca 600
gaggaacagg cctaccatgt cagcaaaggg ctggaatgcc tcaaagccct cagagagaat 660
cctcctgaca ttgaggagat tcaagaggtc agcagcatca gagaccaaac ctgcaaccca 720
ggccaagaga atggaaccac aggcatgcag gaagaggagg actctcagaa tctcgatgaa 780
tcacacgagc caacaaaagg atcaaactat gtcggccatg tactccaaaa taatccggga 840
tgtggagaaa gcaactctgt gtttgtggaa gcagagcagc cccctaaaga ggacatccaa 900
ccaggacctg gaatacgatg ttatcatgtt tatgatcaca gcggtgaaga ggttaaggga 960

atcgaagatg ctgacagtct cgtggtacct gcaggcactg tcggtaatcg aggattcgag 1020
agcggagaag gaagccctga tgatagcact gaggattctg gcgaagatta ttccgaagga 1080
aatgcttcat ctaactgggg atattctttc ggccttaaac cagacagggc agctgatgtg 1140
agcatgccga tggaagagga attaagtgct ctgctcaaga caagcagaaa tgtagggatt 1200
cagaaaaggg atgggaagac tctgcagttc ccacacaatc ccgaaggtaa gacaagggtt 1260
ccggagtgtg gatccatcaa aaaggacaca gaagagaggt cagtctcaca tggaatgggg 1320
atagttgctg gatcgacaag tggtgcaacc caatactagt aattaaaagg gcgaattcc 1379

<210> 16
<211> 21
<212> DNA
<213> Artificial Sequence

<220>
<223> Description of Artificial Sequence: primer

<400> 16
aatgctagag atggtttaat t 21

<210> 17
<211> 19
<212> DNA
<213> Artificial Sequence

<220>
<223> Description of Artificial Sequence: primer

<400> 17
agggctcagg tagtccagc 19

<210> 18
<211> 26
<212> DNA
<213> Artificial Sequence

<220>
<223> Description of Artificial Sequence: primer

<400> 18

catagcaagc caacaggtca accagg 26

<210> 19
<211> 24
<212> DNA
<213> Artificial Sequence

<220>
<223> Description of Artificial Sequence: primer

<400> 19
cctggcaatc aagcgagata tatg 24

<210> 20
<211> 24
<212> DNA
<213> Artificial Sequence

<220>
<223> Description of Artificial Sequence: primer

<400> 20
atgagtgatg gagcagttca acca 24

<210> 21
<211> 26
<212> DNA
<213> Artificial Sequence

<220>
<223> Description of Artificial Sequence: primer

<400> 21
atataatttt ctaggtgcta gttgag 26

<210> 22
<211> 25
<212> DNA

<213> Artificial Sequence

<220>
<223> Description of Artificial Sequence: primer

<400> 22
ggaaagcctg cgcacagccc gaagg                                          25

<210> 23
<211> 29
<212> DNA
<213> Artificial Sequence

<220>
<223> Description of Artificial Sequence: primer

<400> 23
gggacaaaga ggtgtggctt aaatttggg                                      29

<210> 24
<211> 19
<212> DNA
<213> Artificial Sequence

<220>
<223> Description of Artificial Sequence: primer

<400> 24
catcatcaat aattacagg                                                 19

<210> 25
<211> 24
<212> DNA
<213> Artificial Sequence

<220>
<223> Description of Artificial Sequence: primer

<400> 25

cgccggcaca atagtatatt tcat                                          24

<210>  26
<211>  4130
<212>  DNA
<213>  canine parvovirus

<400>  26
gaccgcgtag cgttctggtt gattaagcag agcaaccgac caggccagtc agtctactga      60

tgttgtgcat ctcccaccca cccccccctt aaagacagat tgatacttat ggtaaggtta     120

gttcacctta tagacagtat acgaggccat ttagttttaa atgtctttta ttgatttttt     180

tggtccttaa catattctaa gggcaaacca accaaccacc cacaccataa caacatacat     240

taatggttta caatacattt tataaaacaa actactaact atgatctaaa tgttcttcta     300

tttcttacag ttattgtata ccatataaca aaccttctaa atcctatatc aaatacaagt     360

acaatatttc tatgctgtaa tttaattaat cttaaaataa tatgtaataa gcataaaaac     420

atagtaagta tgttaatata attttctagg tgctagttga gatttttcat atacaatttt     480

catacctcca atattacttg gtacatagtt aaattggtta tctacattaa tactcatttg     540

ttgaattgga ttccaagtat gagaggctct tagtttagct ttaaatacta atttaccttt     600

ccaccaaaaa tctgagtaag ttacaattct tgacatatta gcagatgcat caggatcata     660

ttcatttgtt aaattaggcg caacttttac aaataattga ccaggacaat tattttgaca     720

aacaaatggt gcatttacat gaagtcttgg ttttaagtca gtatcaaatt ctttatccca     780

aatttgacca tttggataaa ctggtggtac attatttaat gcagttaaag gaccataagt     840

attaaatata ttagtatagt taattcctgt tttacctcca attggatctg ttggtagcaa     900

tacattatca tctgttacag gaaggttaaa gttaatattt tgaatccaat ctccttctgg     960

atatcttcct gtatcttgat gtgctatata tgtaaatctc tcaggtgttt ctcctgttgt    1020

ggtagttttt tgaccatgtt gtctaccaaa tgcatatctt ggatcaccat ctgctgcttg 1080

attttcatct gtttgcgctc ccccccgtcc tgctgcaata ggtgttttaa atggcccttg 1140

tgtagacgcc tcaaaagaat aatatggtgc actataacca acctcagctg gtctcataat 1200

agtagcttca gtaatatagt ttgtatttcc catttgagtt acaccacgtc ttttatcttg 1260

ttgaactcct atataaccaa agttagtacc tccttcagat tgaggcaaag aatttagaaa 1320

tggtggtaag cccaatgctc tatttgtttg ccatgtatgt gttagtctac atggtttaca 1380

atcaaaaaaa aatgttcctg tagcaaattc atcacctgtt cttagtaagt gtactggcac 1440

agaattttca atagtataaa attgaacatc atctggatct gtaccatggt atatatttgt 1500

tggtgtgcca ctagttccag tatgagatgg tattaatgtt ctatcccatt gaaaataata 1560

tctccatgga gttggtatgg ttggtttcca tggataaaaa cccaatgtct cagatctcat 1620

agctgctgga gtaaatggca tagtattatt actatctaat gcaaccatca atgatgcagt 1680

taaatcatta ttataaactt tagttggtgg ttgagtagca gattctgaaa cagtctttaa 1740

aacaacatta aaaatttctt gttcaaaact aactaaatgc aactcactca tagtattaac 1800

aattagttgc caatctcctg gattaaacca aactccccaa gcatttgcat caaccaatga 1860

ccaaggtgtt acaatttgtg catgagtatc atctaaagcc atgtttccgt taactgcagt 1920

tttatccaaa ttatttacaa ccactcttct ataattttca ctttctggca tatttaaatg 1980

tacaagtctg cttgagtttg ctgtgatttc cacccatccg ttttccaaaa atttaaattc 2040

cgtctgatta ttgaaagtac ccgtagaaat ccccacaccc ccagaaccac caccaccccc 2100

gcctccagac ccgttcccag atcctgtagc tctttcattt ctgacagcag gttgaccacc 2160

gtctggttga actgctccat cactcattgg tgcaagattg tctcttttta cttgtcctgc 2220

accggctttt tttttttttg caagattgat gaaaatatga ggtggtggtt tacttctttt 2280

agttggtttt gttggtcttg atgttgatgg atgatctggt gtatcagtta atactggagc 2340

aattgccttt ttagctctaa aaaaataatg tcctattttc cccccccaat ctttagcgtc 2400

cttagtttga tctataaagc gttgatctgc tggcgagaaa tataagtatg ggtttttacc 2460

agagcgaaga taagcagcgt aagcttcgtc gtgttctttt gcagcggcgt cagaagggtt 2520

agttggttct ccttggtcaa gactgttccc aggcccaaga tatttataac ctggaggcac 2580

aagtcctata aaaaaacaca tacttagtta agttattaaa tctttcccct ccccccactt 2640

tactaacaca cccttacctc tcctggctct ctttgccgga ggtgccatcg taccttaatc 2700

caagtcgtct cgaaaatctt cttccaattg ttcagaagta aagatggctc tcaggtctgc 2760

ctctatttcg gaccacgtcg gactcgcttg cacgtctttg tgagtaacgc caagttggtt 2820

tgattgttga tttgcagttt ctgcaatagg cgtatctgga gtactccacg gttccagtgc 2880

aaggtccact gcgtccggag tcagaggagt tagaacttgg tcttgactct gaggattgct 2940

tgccgcttgt gtctctaagt ctttgcaacc tggtgaattt ataccttctt gtattttagg 3000

ctccgcccag ttttcatccc attctggtac ttttccccaa tgatgtgtat agttagccat 3060

ggttgattca aaaccatgtt taactaacca tgcacatatt aaaggccatt cttctttatc 3120

aaccaaacca aagtctcctg gaagcttaca tactaactta atgttcaaca ttctgtctct 3180

tattggttgt gtatgttcag gtctttcttc acatccaatt ctcacaattg ttatattttc 3240

attagttgtc ataattactg gagttggttc aatttgctta cttcctttac ctttttgatc 3300

aattctaatt gtttgtccag aacagattgc tttaaattga ttaacttgtt gaccaaagtt 3360

accagcttct tcaatccaaa ttaaattttt attggtacag tcattaaatg gaaaatttac 3420

atttgctgca ttgtaacaac caacattacc cacagcttgt gctatggctt gagcaatgat 3480

agattttcct gtacttgctg gtccatgaaa aagaactgta tttcttttac caccttgtct 3540

atttaaaaca catgctatag cgtgacaaac tttaatccaa ttccatccgt gcattctaaa 3600

aatttgacat gttctagaat ttgcaagatc aaagttagtt agtttagtat tatctgcttt 3660

ttcaagtatt aattcaaatg ctgtttttgt tcttgctaaa gtcaaagtac aaatttcaag 3720

tgtatttttt aaaagatttt cacctcctgg ttgtgccatc atttcaatat aactatctgg 3780

ttgtaacatc atccagtctt caggtgatgt tactctttta ctaaccaagt cccgcaaagt 3840

acatttgatt gacacttcct ttttagtttg aattctcccg cgctttgttt cctgtgctgt 3900

cgtcactgtg gtttcaacgg tttctggttt catttgttca gtgtaaagtg tgctgacaat 3960

ttgtctgtct tgatacttca taaagttaaa tttccaacca gaatcagtac ttaaaaaata 4020

gccactttct tttgtcatgt ggacaatttt tttctttgtt aaaaagtaat atgctatcat 4080

atttccaaaa tgaaccattt taacatagtc ttgacctgca ggcatgcaag 4130